# EUROPEAN PATENT APPLICATION

(11) **EP 4 663 661 A1**
(43) Date of publication of application: **17.12.2025**
(21) Application number: 24752903.5
(22) Date of filing: 07.02.2024
(51) Int. Cl.: C07K 16/28, C12N 15/13, A61K 39/395, A61P 35/00

(54) **ANTI-CDH6 ANTIBODY AND USE THEREOF**

(30) Priority: 10.02.2023 CN 202310109927
(71) Applicant: Hainan Simcere Zaiming Pharmaceutical Co., Ltd., Hainan 570311 (CN)
(72) Inventor: CHAI, Xiaojuan, Shanghai 201318 (CN); FU, Yayuan, Shanghai 201318 (CN); TANG, Renhong, Shanghai 201318 (CN)
(74) Representative: Grünecker Patent- und Rechtsanwälte PartG mbB
(86) International application number: PCT/CN2024/076687
(87) International publication number: WO 2024/165049

(57) **Abstract**

Provided are an antibody specifically binding to cadherin 6 (CDH6) and a use thereof. Specifically disclosed are murine and humanized antibodies binding to CDH6, preparation methods therefor, and uses thereof. The antibodies have good affinity with CDH6 protein and a better endocytic activity, and thus can be applied to the preparation of drugs for treating tumors and the like.

## Description

The present disclosure claims priority to the Chinese Patent Application No. 202310109927.8 entitled "ANTI-CDH6 ANTIBODY AND USE THEREOF", filed with the China National Intellectual Property Administration on February 10, 2023, which is incorporated herein by reference in its entirety.

### TECHNICAL FIELD

The present disclosure relates to the fields of bioengineering and biomedicine, and particularly to an anti-CDH6 antibody or an antigen-binding fragment thereof, a nucleic acid encoding same, an expression vector and an expression cell thereof, a preparation method therefor, a pharmaceutical composition thereof, and the use thereof in treating diseases, for example, the use thereof in treating tumors.

### BACKGROUND

Cadherins play an important role in tissue homeostasis, being primarily responsible for cell-cell adhesion during embryogenesis, tissue morphogenesis, differentiation, and tumorigenesis. Any dysfunction or instability of the cadherin-catenin complex may lead to tumor progression.

CDH6 is a type II classical cadherin, also known as K-cadherin. CDH6 is a single transmembrane protein composed of 790 amino acids, and the extracellular region is divided into 5 regions. It has been found that CDH6 is highly expressed in tumor tissues such as kidney cancer, ovarian cancer, and thyroid cancer, while rarely expressed in normal tissues (Cancer Discov; 2017, 7(9): 1030-45, the content of which is incorporated herein by reference). Like other members of the cadherin superfamily, CDH6 protein localizes to the basolateral membrane of epithelial cells and mediates calcium-dependent cell-cell adhesion with the property of rapid internalization. Therefore, CDH6, as a highly recognizable tumor-specific marker, has become an attractive target in tumor therapy, and can be developed for the treatment of cancers such as ovarian cancer and kidney cancer.

Despite some progress in the treatment of ovarian and kidney cancer in recent years, there is still a substantial unmet clinical need. Currently, there are few CDH6-targeted drugs developed, and no related drugs have been approved for marketing. Therefore, drugs developed for this target have broad clinical application and treatment prospects.

### SUMMARY

The present disclosure provides an antibody or an antigen-binding fragment specifically binding to CDH6, nucleic acids encoding the antibodies and the antigen-binding fragments thereof, and a pharmaceutical composition and a kit comprising the antibody and the antigen-binding fragment, which can be used for preparation of a drug for treating a tumor and the like.

In a first aspect, the present disclosure provides an antibody or an antigen-binding fragment specifically binding to CDH6, wherein the antibody or the antigen-binding fragment specifically binds to one or more amino acid sequence fragments of EC1 region, EC1-EC2 region, EC2 region, EC2-EC3 region, EC3 region, EC4 region, or EC5 region of an extracellular region of CDH6 protein; preferably, the antibody or the antigen-binding fragment specifically binds to one or more amino acid sequence fragments set forth in any one sequence of SEQ ID NOs: 168-172; preferably, the antibody or the antigen-binding fragment has endocytic activity.

In a second aspect, the present disclosure provides an antibody or an antigen-binding fragment specifically binding to CDH6, wherein the antibody or the antigen-binding fragment comprises:
(a) an HCDR1, an HCDR2, and an HCDR3 of the VH set forth in any one of SEQ ID NOs: 2, 4, 6, 8, 10, 12, 14, 16, 18, 20, 22, 24, 26, 28, 136, 137, 138, 139, 150, 151, 152, 160, 161, 162, 163, 164, and 165, and/or (b) an LCDR1, an LCDR2, and an LCDR3 of the VL set forth in any one of SEQ ID NOs: 3, 5, 7, 9, 11, 13, 15, 17, 19, 21, 23, 25, 27, 29, 133, 134, 135, 145, 146, 147, 148, 149, 157, 158, and 159;
preferably, the HCDR1-3 and/or the LCDR1-3 are encoded according to a universal analysis method of KABAT, IMGT, or Chothia.

In a specific embodiment, the HCDR1, the HCDR2 and the HCDR3 have a sequence combination selected from any of the following sequence combinations or a sequence combination with 1, 2, 3, or more amino acid insertions, deletions, and/or substitutions compared with the sequence combinations:

| Sequence No. | SEQ ID NO. | | |
|---|---|---|---|
| | HCDR1 | HCDR2 | HCDR3 |
| VH1 | SEQ ID NO.36 | SEQ ID NO.37 | SEQ ID NO.38 |
| VH2 | SEQ ID NO.42 | SEQ ID NO.43 | SEQ ID NO.44 |
| VH3 | SEQ ID NO.48 | SEQ ID NO.49 | SEQ ID NO.50 |
| VH4 | SEQ ID NO.54 | SEQ ID NO.55 | SEQ ID NO.56 |
| VH5 | SEQ ID NO.60 | SEQ ID NO.61 | SEQ ID NO.62 |
| VH6 | SEQ ID NO.66 | SEQ ID NO.67 | SEQ ID NO.68 |
| VH7 | SEQ ID NO.72 | SEQ ID NO.73 | SEQ ID NO.74 |
| VH8 | SEQ ID NO.78 | SEQ ID NO.79 | SEQ ID NO.80 |
| VH9 | SEQ ID NO.84 | SEQ ID NO.85 | SEQ ID NO.86 |
| VH10 | SEQ ID NO.90 | SEQ ID NO.91 | SEQ ID NO.92 |
| VH11 | SEQ ID NO.96 | SEQ ID NO.97 | SEQ ID NO.98 |
| VH12 | SEQ ID NO.102 | SEQ ID NO.103 | SEQ ID NO.104 |
| VH13 | SEQ ID NO.108 | SEQ ID NO.109 | SEQ ID NO.110 |
| VH14 | SEQ ID NO.114 | SEQ ID NO.115 | SEQ ID NO.116 |

and,
the LCDR1, the LCDR2 and the LCDR3 have a sequence combination selected from any of the following sequence combinations or a sequence combination with 1, 2, 3, or more amino acid insertions, deletions, and/or substitutions compared with the sequence combinations:

| Sequence No. | SEQ ID NO. | | |
|---|---|---|---|
| | LCDR1 | LCDR2 | LCDR3 |
| VL1 | SEQ ID NO.39 | SEQ ID NO.40 | SEQ ID NO.41 |
| VL2 | SEQ ID NO.45 | SEQ ID NO.46 | SEQ ID NO.47 |
| VL3 | SEQ ID NO.51 | SEQ ID NO.52 | SEQ ID NO.53 |
| VL4 | SEQ ID NO.57 | SEQ ID NO.58 | SEQ ID NO.59 |
| VL5 | SEQ ID NO.63 | SEQ ID NO.64 | SEQ ID NO.65 |
| VL6 | SEQ ID NO.69 | SEQ ID NO.70 | SEQ ID NO.71 |
| VL7 | SEQ ID NO.75 | SEQ ID NO.76 | SEQ ID NO.77 |
| VL8 | SEQ ID NO.81 | SEQ ID NO.82 | SEQ ID NO.83 |
| VL9 | SEQ ID NO.87 | SEQ ID NO.88 | SEQ ID NO.89 |
| VL10 | SEQ ID NO.93 | SEQ ID NO.94 | SEQ ID NO.95 |
| VL11 | SEQ ID NO.99 | SEQ ID NO.100 | SEQ ID NO.101 |
| VL12 | SEQ ID NO.105 | SEQ ID NO.106 | SEQ ID NO.107 |
| VL13 | SEQ ID NO.111 | SEQ ID NO.112 | SEQ ID NO.113 |
| VL14 | SEQ ID NO.117 | SEQ ID NO.118 | SEQ ID NO.119 |

preferably, the substitutions are conservative amino acid substitutions.

In another specific embodiment, the antibody or the antigen-binding fragment comprises a combination of heavy chain CDRs and light chain CDRs selected from: VH1+VL1, VH2+VL2, VH3+VL3, VH4+VL4, VH5+VL5, VH6+VL6, VH7+VL7, VH8+VL8, VH9+VL9, VH10+VL10, VH11+VL11, VH12+VL12, VH13+VL13, and VH14+VL14, and a combination of CDRs with 1, 2, 3 or more amino acid insertions, deletions, and/or substitutions compared with the sequences of the combination of the heavy chain and light chain CDRs, wherein
preferably, the substitutions are conservative amino acid substitutions.

In another specific embodiment, framework regions of the heavy chain variable region and the light chain variable region of the antibody or the antigen-binding fragment are derived from a human germline heavy chain template and a human germline light chain template, wherein:
(1) the framework region sequences are derived from a combined sequence of a human germline heavy chain IGHV1-69*02 and IGHJ6*01, comprising FR1, FR2, and FR3 regions of IGHV1-69*02 set forth in SEQ ID NO: 143 and an FR4 region of IGHJ6*01 set forth in SEQ ID NO: 144;
(2) the framework region sequences are derived from a combined sequence of a human germline heavy chain IGHV1-46*01 and IGHJ6*01, comprising FR1, FR2, and FR3 regions of IGHV1-46*01 set forth in SEQ ID NO: 156 and an FR4 region of IGHJ6*01 set forth in SEQ ID NO: 144;
(3) the framework region sequences are derived from a combined sequence of a human germline heavy chain IGHV1-3*01 and IGHJ6*01, comprising FR1, FR2, and FR3 regions of IGHV1-3*01 set forth in SEQ ID NO: 166 and an FR4 region of IGHJ6*01 set forth in SEQ ID NO: 144;
(4) the framework region sequences are derived from a combined sequence of a human germline light chain IGKV4-1*01 and IGKJ4*01, comprising FR1, FR2, and FR3 regions of IGKV4-1*01 set forth in SEQ ID NO: 140 and an FR4 region of IGKJ4*01 set forth in SEQ ID NO: 142;
(5) the framework region sequences are derived from a combined sequence of a human germline light chain IGKV1-33*01 and IGKJ4*01, comprising FR1, FR2, and FR3 regions of IGKV1-33*01 set forth in SEQ ID NO: 141 and an FR4 region of IGKJ4*01 set forth in SEQ ID NO: 142;
(6) the framework region sequences are derived from a combined sequence of a human germline light chain IGKV1-NL1*01 and IGKJ2*01, comprising FR1, FR2, and FR3 regions of IGKV1-NL1*01 set forth in SEQ ID NO: 153 and an FR4 region of IGKJ2*01 set forth in SEQ ID NO: 155; or
(7) the framework region sequences are derived from a combined sequence of a human germline light chain IGKV2-28*01 and IGKJ2*01, comprising FR1, FR2, and FR3 regions of IGKV2-28*01 set forth in SEQ ID NO: 154 and an FR4 region of IGKJ2*01 set forth in SEQ ID NO: 155.

In a specific embodiment, the framework regions of the antibody or the antigen-binding fragment, according to numbers of the Kabat numbering scheme, further comprise one or more mutations selected from the following group, wherein
(1) the framework regions of the heavy chain variable region comprise: G27Y, S30T, A40R, I70L, or A72V, preferably comprise G27Y and A72V, or preferably comprise G27Y, S30T, and A72V, or preferably comprise G27Y, S30T, I70L, and A72V, or preferably comprise G27Y, S30T, A40R, and A72V;
(2) the framework regions of the heavy chain variable region comprise: G42R, M70L, R72V, or T74K, preferably comprise R72V and T74K, or preferably comprise M70L, R72V, and T74K, or preferably comprise G42R, R72V, and T74K;
(3) the framework regions of the heavy chain variable region comprise: V2I, V5Q, R44G, I70L, R72V, Y95L, or R98S, preferably comprise R72V and R98S, or preferably comprise V2I, R72V, and R98S, or preferably comprise V2I, I70L, R72V, and R98S, or preferably comprise R44G, R72V, and R98S, or preferably comprise V5Q, R72V, and R98S, or preferably comprise R72V, Y95L, and R98S;
(4) the framework regions of the light chain variable region comprise: P44S, A47P, or G72E, preferably comprise G72E, or preferably comprise P44S and G72E, or preferably comprise A47P and G72E;
(5) the framework regions of the light chain variable region comprise: Q42K, A43S, K45Q, L48V, I48V, or T85R, preferably comprise A43S, or preferably comprise A43S and L48V, or preferably comprise A43S, K45Q, and L48V, or preferably comprise A43S, L48V, and T85R, or preferably comprise Q42K and I48V; or
(6) the framework regions of the light chain variable region comprise: K42G, P43T, P44F, or Y49S, preferably comprise P44F and Y49S, or preferably comprise K42G and Y49S, or preferably comprise P43T and Y49S.

In a specific embodiment, the antibody or the antigen-binding fragment comprises:
(1) a heavy chain variable region having the sequence set forth in SEQ ID NO: 2, 4, 6, 8, 10, 12, 14, 16, 18, 20, 22, 24, 26, 28, 136, 137, 138, 139, 150, 151, 152, 160, 161, 162, 163, 164, or 165;
(2) a light chain variable region having the sequence set forth in SEQ ID NO: 3, 5, 7, 9, 11, 13, 15, 17, 19, 21, 23, 25, 27, 29, 133, 134, 135, 145, 146, 147, 148, 149, 157, 158, or 159;
(3) an amino acid sequence having at least 90% identity, preferably at least 91%, 92%, 93%, 94%, 95%, 96%, 97%, 98%, or 99% identity, to any one of the sequences set forth in (1) to (2) above.

In another specific embodiment, the heavy chain variable region and the light chain variable region are selected from the following group:
(1) having the VH set forth in SEQ ID NO. 2 and the VL set forth in SEQ ID NO. 3;
(2) having the VH set forth in SEQ ID NO. 4 and the VL set forth in SEQ ID NO. 5;
(3) having the VH set forth in SEQ ID NO. 6 and the VL set forth in SEQ ID NO. 7;
(4) having the VH set forth in SEQ ID NO. 8 and the VL set forth in SEQ ID NO. 9;
(5) having the VH set forth in SEQ ID NO. 10 and the VL set forth in SEQ ID NO. 11;
(6) having the VH set forth in SEQ ID NO. 12 and the VL set forth in SEQ ID NO. 13;
(7) having the VH set forth in SEQ ID NO. 14 and the VL set forth in SEQ ID NO. 15;
(8) having the VH set forth in SEQ ID NO. 16 and the VL set forth in SEQ ID NO. 17;
(9) having the VH set forth in SEQ ID NO. 18 and the VL set forth in SEQ ID NO. 19;
(10) having the VH set forth in SEQ ID NO. 20 and the VL set forth in SEQ ID NO. 21;
(11) having the VH set forth in SEQ ID NO. 22 and the VL set forth in SEQ ID NO. 23;
(12) having the VH set forth in SEQ ID NO. 24 and the VL set forth in SEQ ID NO. 25;
(13) having the VH set forth in SEQ ID NO. 26 and the VL set forth in SEQ ID NO. 27;
(14) having the VH set forth in SEQ ID NO. 28 and the VL set forth in SEQ ID NO. 29;
(15) having the VH set forth in SEQ ID NO. 136 and the VL set forth in SEQ ID NO. 133;
(16) having the VH set forth in SEQ ID NO. 137 and the VL set forth in SEQ ID NO. 133;
(17) having the VH set forth in SEQ ID NO. 138 and the VL set forth in SEQ ID NO. 133;
(18) having the VH set forth in SEQ ID NO. 139 and the VL set forth in SEQ ID NO. 133;
(19) having the VH set forth in SEQ ID NO. 136 and the VL set forth in SEQ ID NO. 134;
(20) having the VH set forth in SEQ ID NO. 137 and the VL set forth in SEQ ID NO. 134;
(21) having the VH set forth in SEQ ID NO. 138 and the VL set forth in SEQ ID NO. 134;
(22) having the VH set forth in SEQ ID NO. 139 and the VL set forth in SEQ ID NO. 134;
(23) having the VH set forth in SEQ ID NO. 136 and the VL set forth in SEQ ID NO. 135;
(24) having the VH set forth in SEQ ID NO. 137 and the VL set forth in SEQ ID NO. 135;
(25) having the VH set forth in SEQ ID NO. 138 and the VL set forth in SEQ ID NO. 135;
(26) having the VH set forth in SEQ ID NO. 139 and the VL set forth in SEQ ID NO. 135;
(27) having the VH set forth in SEQ ID NO. 150 and the VL set forth in SEQ ID NO. 145;
(28) having the VH set forth in SEQ ID NO. 150 and the VL set forth in SEQ ID NO. 146;
(29) having the VH set forth in SEQ ID NO. 150 and the VL set forth in SEQ ID NO. 147;
(30) having the VH set forth in SEQ ID NO. 150 and the VL set forth in SEQ ID NO. 148;
(31) having the VH set forth in SEQ ID NO. 150 and the VL set forth in SEQ ID NO. 149;
(32) having the VH set forth in SEQ ID NO. 151 and the VL set forth in SEQ ID NO. 145;
(33) having the VH set forth in SEQ ID NO. 151 and the VL set forth in SEQ ID NO. 146;
(34) having the VH set forth in SEQ ID NO. 151 and the VL set forth in SEQ ID NO. 147;
(35) having the VH set forth in SEQ ID NO. 151 and the VL set forth in SEQ ID NO. 148;
(36) having the VH set forth in SEQ ID NO. 151 and the VL set forth in SEQ ID NO. 149;
(37) having the VH set forth in SEQ ID NO. 152 and the VL set forth in SEQ ID NO. 145;
(38) having the VH set forth in SEQ ID NO. 152 and the VL set forth in SEQ ID NO. 146;
(39) having the VH set forth in SEQ ID NO. 152 and the VL set forth in SEQ ID NO. 147;
(40) having the VH set forth in SEQ ID NO. 152 and the VL set forth in SEQ ID NO. 148;
(41) having the VH set forth in SEQ ID NO. 152 and the VL set forth in SEQ ID NO. 149;
(42) having the VH set forth in SEQ ID NO. 160 and the VL set forth in SEQ ID NO. 157;
(43) having the VH set forth in SEQ ID NO. 160 and the VL set forth in SEQ ID NO. 158;
(44) having the VH set forth in SEQ ID NO. 160 and the VL set forth in SEQ ID NO. 159;
(45) having the VH set forth in SEQ ID NO. 161 and the VL set forth in SEQ ID NO. 157;
(46) having the VH set forth in SEQ ID NO. 161 and the VL set forth in SEQ ID NO. 158;
(47) having the VH set forth in SEQ ID NO. 161 and the VL set forth in SEQ ID NO. 159;
(48) having the VH set forth in SEQ ID NO. 162 and the VL set forth in SEQ ID NO. 157;
(49) having the VH set forth in SEQ ID NO. 162 and the VL set forth in SEQ ID NO. 158;
(50) having the VH set forth in SEQ ID NO. 162 and the VL set forth in SEQ ID NO. 159;
(51) having the VH set forth in SEQ ID NO. 163 and the VL set forth in SEQ ID NO. 157;
(52) having the VH set forth in SEQ ID NO. 163 and the VL set forth in SEQ ID NO. 158;
(53) having the VH set forth in SEQ ID NO. 163 and the VL set forth in SEQ ID NO. 159;
(54) having the VH set forth in SEQ ID NO. 164 and the VL set forth in SEQ ID NO. 157;
(55) having the VH set forth in SEQ ID NO. 164 and the VL set forth in SEQ ID NO. 158;
(56) having the VH set forth in SEQ ID NO. 164 and the VL set forth in SEQ ID NO. 159;
(57) having the VH set forth in SEQ ID NO. 165 and the VL set forth in SEQ ID NO. 157;
(58) having the VH set forth in SEQ ID NO. 165 and the VL set forth in SEQ ID NO. 158;
(59) having the VH set forth in SEQ ID NO. 165 and the VL set forth in SEQ ID NO. 159; or
(60) a combination of a VH and a VL having at least 90%, 91%, 92%, 93%, 94%, 95%, 96%, 97%, 98%, 99%, or 100% sequence identity to any one of the sequence combinations (1) to (59) above. In a specific embodiment, the antibody or the antigen-binding fragment binds to human CDH6 protein with a dissociation constant (KD) not greater than 2×10⁻⁷ M.

In another specific embodiment, the antibody or the antigen-binding fragment is:
(1) a chimeric antibody or a fragment thereof;
(2) a humanized antibody or a fragment thereof;
(3) a fully human antibody or a fragment thereof;
preferably, the antibody or the antigen-binding fragment is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', a F(ab')2, an Fd, an Fv, an scFv, a diabody, and a single domain antibody.

In another specific embodiment, the antibody comprises a sequence of a constant region of any one of human or murine antibodies IgG1, IgG2, IgG3, IgG4, IgA, IgM, IgE, and IgD, and preferably comprises a sequence of a constant region of human or murine antibody IgG1, IgG2, IgG3, or IgG4. In another specific embodiment, the antigen-binding fragment is selected from one or more of a F(ab)2, a Fab', a Fab, an Fv, an scFv, a bispecific antibody, a nanobody, and a minimal recognition unit of an antibody.

In a third aspect, the antibody or the antigen-binding fragment of the present disclosure is further conjugated to a therapeutic agent or a tracer; preferably, the therapeutic agent is selected from a radioisotope, a chemotherapeutic agent, and an immunomodulator, and the tracer is selected from a radiocontrast medium, a paramagnetic ion, a metal, a fluorescent label, a chemiluminescent label, an ultrasound contrast agent, and a photosensitizer.

In a fourth aspect, the present disclosure further provides a multispecific antigen-binding molecule, which comprises a first antigen-binding moiety and a second antigen-binding moiety, wherein the first antigen-binding moiety comprises the antibody or the antigen-binding fragment according to any one of the first aspect and the second aspect above, and the second antigen-binding moiety specifically binds to an antigen other than CDH6 or to a CDH6 epitope different from the first antigen-binding moiety, wherein
preferably, the antigen is selected from CD3, CD7, CD16, CD16A, CD4, CD5, CD8, CD14, CD15, CD19, CD20, CD21, CD23, CD25, CD33, CD37, CD38, CD40, CD40L, CD46, CD52, CD54, CD66(a-d), CD74, CD80, CD126, CD138, BCMA, HLA-DR, HER2, VEGF, P1GF, HER3/ERBB3, HER4/ERBB4, IL-2, IL-6, PD-1, PD-L1, TRAIL-R1, and TRAIL-R2;
preferably, the multispecific antigen-binding molecule is a bispecific antibody, a trispecific antibody, or a tetraspecific antibody.

In a fifth aspect, the present disclosure further provides a chimeric antigen receptor (CAR), wherein the chimeric antigen receptor at least comprises an extracellular antigen-binding domain, a transmembrane domain, and an intracellular signaling domain; the extracellular antigen-binding domain comprises the CDH6 antibody or the antigen-binding fragment according to the first aspect or the second aspect above.

In a sixth aspect, the present disclosure further provides an immune effector cell comprising the chimeric antigen receptor according to the fifth aspect described above or comprising a nucleic acid fragment encoding the chimeric antigen receptor according to the fifth aspect described above;
preferably, the immune effector cell is selected from a T cell, an NK cell (natural killer cell), an NKT cell (natural killer T cell), a monocyte, a macrophage, a dendritic cell, and a mast cell, wherein the T cell can be selected from an inflammatory T cell, a cytotoxic T cell, a regulatory T cell (Treg), and a helper T cell; and
preferably, the immune effector cell is an allogeneic immune effector cell or an autologous immune cell.

In a seventh aspect, the present disclosure further provides an isolated nucleic acid molecule encoding the antibody, the antigen-binding fragment, or any combination thereof according to the first and second aspects described above, the antibody or the antigen-binding fragment conjugated to a tracer according to the third aspect, the multispecific antigen-binding molecule according to the fourth aspect, or the chimeric antigen receptor according to the fifth aspect.

In an eighth aspect, the present disclosure further provides an expression vector comprising the isolated nucleic acid molecule according to the seventh aspect.

In a ninth aspect, the present disclosure provides an isolated host cell of the isolated nucleic acid molecule according to the seventh aspect above or the expression vector according to the eighth aspect; preferably, the host cell is a eukaryotic cell or a prokaryotic cell; more preferably, the host cell is derived from a mammalian cell, a yeast cell, an insect cell, *E. coli,* and/or *Bacillus subtilis;* and more preferably, the host cell is selected from Expi293 and CHO cells.

In a tenth aspect, the present disclosure provides a method for preparing the antibody or the antigen-binding fragment according to the first or second aspect, the antibody or the antigen-binding fragment conjugated to a tracer according to the third aspect, or the multispecific antigen-binding molecule according to the fourth aspect above, wherein the method comprises culturing the host cell according to the ninth aspect under appropriate conditions, and isolating the antibody or the antigen-binding fragment or the multispecific antigen-binding molecule.

In an eleventh aspect, the present disclosure provides a method for preparing the immune effector cell according to the sixth aspect above, wherein the method comprises introducing a nucleic acid fragment encoding the CAR according to the fifth aspect into the immune effector cell; optionally, the method further comprises initiating expression of the CAR according to the fifth aspect in the immune effector cell.

In a twelfth aspect, the present disclosure provides a pharmaceutical composition comprising the antibody or the antigen-binding fragment according to the first or second aspect, the antibody or the antigen-binding fragment conjugated to a tracer according to the third aspect, the multispecific antigen-binding molecule according to the fourth aspect, the chimeric antigen receptor according to the fifth aspect, the immune effector cell according to the sixth aspect, the isolated nucleic acid molecule according to the seventh aspect, the expression vector according to the eighth aspect, the cell according to the ninth aspect, or a product prepared by the method according to the tenth or eleventh aspect above, wherein preferably, the composition further comprises a pharmaceutically acceptable carrier, diluent, or adjuvant; preferably, the pharmaceutical composition further comprises an additional antineoplastic agent.

In a thirteenth aspect, the present disclosure provides use of the antibody or the antigen-binding fragment according to the first or second aspect, the antibody or the antigen-binding fragment conjugated to a tracer according to the third aspect, the multispecific antigen-binding molecule according to the fourth aspect, the chimeric antigen receptor according to the fifth aspect, the immune effector cell according to the sixth aspect, the isolated nucleic acid molecule according to the seventh aspect, the expression vector according to the eighth aspect, the cell according to the ninth aspect, or a product prepared by the method according to the tenth or eleventh aspect, or the pharmaceutical composition according to the twelfth aspect above in preparing a medicament for preventing and/or treating a tumor disease; preferably, the tumor disease is selected from a solid tumor; preferably, the solid tumor is a solid tumor expressing CDH6 protein; more preferably, the solid tumor is selected from kidney cancer, ovarian cancer, thyroid cancer, cholangiocarcinoma, lung cancer, glioblastoma, mesothelioma, uterine cancer, pancreatic cancer, Wilms tumor, and neuroblastoma.

In a fourteenth aspect, the present disclosure provides a method for preventing and/or treating a tumor disease, comprising administering to a patient in need thereof an effective amount of the antibody or the antigen-binding fragment according to the first or second aspect, the antibody or the antigen-binding fragment conjugated to a tracer according to the third aspect, the multispecific antigen-binding molecule according to the fourth aspect, the chimeric antigen receptor according to the fifth aspect, the immune effector cell according to the sixth aspect, the isolated nucleic acid molecule according to the seventh aspect, the expression vector according to the eighth aspect, the cell according to the ninth aspect, or a product prepared by the method according to the tenth or eleventh aspect, or the pharmaceutical composition according to the twelfth aspect above; preferably, the tumor disease is selected from a solid tumor; preferably, the solid tumor is a solid tumor expressing CDH6 protein; more preferably, the solid tumor is selected from kidney cancer, ovarian cancer, thyroid cancer, cholangiocarcinoma, lung cancer, glioblastoma, mesothelioma, uterine cancer, pancreatic cancer, Wilms tumor, and neuroblastoma.

In a fifteenth aspect, the present disclosure provides the antibody or the antigen-binding fragment according to the first or second aspect, the antibody or the antigen-binding fragment conjugated to a tracer according to the third aspect, the multispecific antigen-binding molecule according to the fourth aspect, the chimeric antigen receptor according to the fifth aspect, the immune effector cell according to the sixth aspect, the isolated nucleic acid molecule according to the seventh aspect, the expression vector according to the eighth aspect, the cell according to the ninth aspect, or a product prepared by the method according to the tenth or eleventh aspect, or the pharmaceutical composition according to the twelfth aspect above for use in preventing and/or treating a tumor disease; preferably, the tumor disease is selected from a solid tumor; preferably, the solid tumor is a solid tumor expressing CDH6 protein; more preferably, the solid tumor is selected from kidney cancer, ovarian cancer, thyroid cancer, cholangiocarcinoma, lung cancer, glioblastoma, mesothelioma, uterine cancer, pancreatic cancer, Wilms tumor, and neuroblastoma.

In a sixteenth aspect, the present disclosure provides a kit comprising the antibody or the antigen-binding fragment according to the first or second aspect, the antibody or the antigen-binding fragment conjugated to a tracer according to the third aspect, the multispecific antigen-binding molecule according to the fourth aspect, the chimeric antigen receptor according to the fifth aspect, the immune effector cell according to the sixth aspect, the isolated nucleic acid molecule according to the seventh aspect, the expression vector according to the eighth aspect, the cell according to the ninth aspect, or a product prepared by the method according to the tenth or eleventh aspect, or the pharmaceutical composition according to the twelfth aspect above, and instructions.

Beneficial effects: The present disclosure aims to develop a monoclonal antibody specifically binding to CDH6, which has good binding activity and internalization capability, can be used for the subsequent development of drugs such as CDH6-ADC, and is used for treating diseases such as ovarian cancer and kidney cancer.

### TERMINOLOGY AND DEFINITIONS

As used herein, the term "antibody" (Ab) refers to an immunoglobulin molecule that specifically binds to a target antigen or has immunoreactivity, including polyclonal, monoclonal, genetically engineered and other modified forms of antibodies (including, but not limited to, chimeric antibodies, humanized antibodies, fully human antibodies, heteroconjugated antibodies (e.g., bispecific, trispecific and tetraspecific antibodies, diabodies, triabodies, tetrabodies, and antibody conjugates)), and antigen-binding fragments of antibodies (including, for example, Fab', F(ab')2, Fab, Fv, rIgG, and scFv fragments). Furthermore, unless otherwise indicated, the term "monoclonal antibody" (mAb) is intended to include intact antibody molecules and incomplete antibody fragments (e.g., Fab and F(ab')2 fragments which are short of the Fc fragment of an intact antibody (cleared more rapidly from the animal circulation) and thus are short of Fc-mediated effector function (see Wahl et al., J.Nucl.Med. 24: 316, 1983, the content of which is incorporated herein by reference)) capable of specifically binding to a target protein.

The "antibody" herein may be derived from any animal, including, but not limited to, human and non-human animals which may be selected from primates, mammals, rodents, and vertebrates, such as Camelidae species, *Lama glama, Lama guanicoe, Vicugna pacos,* sheep, rabbits, mice, rats, or Chondrichthyes species (e.g., shark).

The term "monospecific" herein means having one or more binding sites, each of which binds to the same epitope of the same antigen.

The term "multispecific" herein means having at least two antigen-binding sites, each of which binds to a different epitope of the same antigen or a different epitope of a different antigen. Thus, the terms such as "bispecific", "trispecific", and "tetraspecific" refer to the number of different epitopes to which an antibody/antigen-binding molecule can bind.

"Full-length antibody", "complete antibody", and "intact antibody" herein are used interchangeably and refer to an antibody having a substantially similar structure to a natural antibody.

As used herein, the term "antigen-binding fragment" refers to one or more fragments of an antibody that retain the ability to specifically bind to a target antigen. The antigen-binding function of an antibody may be performed by fragments of a full-length antibody. An antibody fragment may be a Fab, F(ab')2, scFv, SMIP, diabody, triabody, affibody, nanobody, aptamer, or domain antibody. Examples of binding fragments encompassing the term "antigen-binding fragment" of an antibody include, but are not limited to: (i) a Fab fragment, i.e., a monovalent fragment consisting of VL, VH, CL and CH1 domains; (ii) an F(ab)2 fragment, i.e., a bivalent fragment comprising two Fab fragments linked by a disulfide bond at the hinge region; (iii) an Fd fragment consisting of VH and CH1 domains; (iv) an Fv fragment consisting of VL and VH domains of a single arm of an antibody; (V) a dAb comprising VH and VL domains; (vi) a dAb fragment consisting of a VH domain (Ward et al., Nature 341: 544-546, 1989, the content of which is incorporated herein by reference); (vii) a dAb consisting of a VH or VL domain; (viii) an isolated complementarity determining region (CDR); and (ix) a combination of two or more isolated CDRs, which may optionally be joined by a synthetic linker. Furthermore, although the two domains (VL and VH) of the Fv fragment are encoded by separate genes, these two domains may be joined using a recombination method through a linker that enables them to be made into a single protein chain in which the VL and VH regions are paired to form a monovalent molecule (known as single chain Fv (scFv); see, e.g., Bird et al., Science 242: 423-426, 1988 and Huston et al., Proc. Natl. Acad. Sci. USA 85: 5879-5883, 1988, the content of which is incorporated herein by reference). These antibody fragments may be obtained using a conventional technique known to those skilled in the art, and these fragments are screened for use in the same manner as intact antibodies. Antigen-binding fragments may be produced by a recombinant DNA technique or enzymatic or chemical cleavage of intact immunoglobulins or, in some embodiments, by a chemical peptide synthesis procedure known in the art.

As used herein, the term "CDH6", also known as "cadherin 6", refers to a cell adhesion molecule that is a member of the cadherin family of cell-cell adhesion molecules. CDH6 is a single transmembrane protein composed of 790 amino acids, which is classified into the type II cadherin family, and it has an N-terminal extracellular and a C-terminal intracellular domain. The CDH6 protein used in the present disclosure can be directly purified from CDH6-expressing cells of a human or non-human mammal (e.g., rat, mouse, or monkey) and can then be used, or a cell membrane fraction of the above cells can be prepared, which can be used as CDH6 protein. Alternatively, CDH6 may be obtained by allowing a host cell to produce CDH6 by *in vitro* synthesis or by genetic manipulation. According to such genetic manipulation, CDH6 protein can be obtained. Specifically, CDH6 cDNA is incorporated into a vector capable of expressing CDH6 cDNA, and then CDH6 is synthesized in a solution containing enzymes, substrates, and energy materials required for transcription and translation, or by transforming host cells of other prokaryotes or eukaryotes, thereby allowing them to express CDH6. CDH6-expressing cells or CDH6-expressing cell lines based on the above genetic manipulations can also be used to present CDH6 protein. Alternatively, an expression vector that has incorporated CDH6 cDNA can be administered directly to the animal to be immunized, and CDH6 can be expressed in the animal immunized as described.

As used herein, the term "bispecific antibody" refers to an antibody, typically a human or humanized antibody, having monoclonal binding specificity for at least two different antigens. In the present disclosure, one of the binding specificities can be detected for an antigenic epitope of CDH6, and the other can be detected for another antigenic epitope of CDH6 or any other antigen other than CDH6, e.g., for a cell surface protein, a receptor, a receptor subunit, a tissue specific antigen, a virus-derived protein, a virus-encoded envelope protein, a bacterial-derived protein, or a bacterial surface protein, etc.

As used herein, the term "chimeric" antibody refers to an antibody having a variable sequence of an immunoglobulin derived from one source organism (e.g., rat or mouse) and a constant region of an immunoglobulin derived from a different organism (e.g., human). Methods for producing chimeric antibodies are known in the art.

As used herein, the term "complementarity determining region" (CDR) refers to a hypervariable region found in both light and heavy chain variable domains. The highly conserved portions of the variable domains are called framework regions (FRs). As known in the art, the amino acid positions representing the hypervariable regions of an antibody may vary according to the context and various definitions known in the art. Some positions within a variable domain may be considered to be hybrid hypervariable positions in that such positions may be considered to be within a hypervariable region according to one set of standards (such as IMGT or KABAT) but outside a hypervariable region according to a different set of standards (such as KABAT or IMGT). One or more of such positions may also be found in extended hypervariable regions. The present disclosure includes antibodies comprising modifications in such hybrid hypervariable positions. The variable domains of native heavy and light chains each comprise four framework regions, mainly in a sheet configuration, connected by three CDRs (CDR1, CDR2, and CDR3) that form loops connecting the sheet structures, and in some cases form part of the sheet structures. The CDRs in each chain are held tightly together by the FR regions, in the order of FR1-CDR1-FR2-CDR2-FR3-CDR3-FR4, and contribute to the formation of the antigen-binding site of the antibody with CDRs from other antibody chains (see Kabat et al., Sequences of Proteins of Immunological Interest, National Institute of Health, Bethesda, Md. 1987, which is incorporated herein by reference). For example, CDR1-VH, CDR2-VH, and CDR3-VH herein refer to the first CDR, second CDR, and third CDR, respectively, of the heavy chain variable region (VH), which constitute a CDR combination (VHCDR combination) of the heavy chain (or the variable region thereof); CDR1-VL, CDR2-VL, and CDR3-VL refer to the first CDR, second CDR, and third CDR, respectively, of the light chain variable region (VL), which constitute a CDR combination (VLCDR combination) of the light chain (or the variable region thereof).

As used herein, the term "monoclonal antibody" refers to an antibody derived from a single clone (including any eukaryotic, prokaryotic, or phage clone), and is not limited to the production method of the antibody.

As used herein, the term "VH" refers to the variable region of an immunoglobulin heavy chain of an antibody (including the heavy chain of an Fv, an scFv, or a Fab). The term "VL" refers to the variable region of an immunoglobulin light chain (including the light chain of an Fv, an scFv, a dsFv, or a Fab).

The term "heavy chain constant region" herein refers to the carboxyl-terminal portion of an antibody heavy chain that is not directly involved in the binding of the antibody to an antigen, but exhibits effector functions, such as interaction with an Fc receptor; the heavy chain constant region has a more conserved amino acid sequence relative to the variable domain of the antibody. The "heavy chain constant region" comprises at least one of a CH1 domain, a hinge region, a CH2 domain, a CH3 domain, or a variant or fragment thereof. The "heavy chain constant region" includes a "full-length heavy chain constant region" having a structure substantially similar to that of a natural antibody constant region and a "heavy chain constant region fragment" including only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; when the antibody is a heavy chain antibody, it does not comprise a CH1 domain. Illustratively, a typical "heavy chain constant region fragment" may be selected from CH1, Fc, and CH3 domains.

The term "light chain constant region" herein refers to the carboxyl-terminal portion of an antibody light chain that is not directly involved in the binding of the antibody to an antigen. The light chain constant region may be selected from a constant κ domain and a constant λ domain.

The term "Fc" herein refers to the carboxyl-terminal portion of an antibody that is formed by the hydrolysis of an intact antibody by papain, which typically comprises the CH3 and CH2 domains of the antibody. The Fc region includes, for example, an Fc region of native sequences, a recombinant Fc region, and a variant Fc region. Although the boundaries of the Fc region of an immunoglobulin heavy chain may vary slightly, the human IgG heavy chain Fc region is generally defined to stretch from an amino acid residue at position Cys226, or from Pro230, to the carboxyl terminus thereof. The C-terminal lysine of the Fc region (residue 447 according to the EU numbering scheme) may be removed, for example, during production or purification of the antibody, or by recombinant engineering of the nucleic acid encoding the heavy chain of the antibody, and thus, the Fc region may or may not include Lys447.

The term "humanized antibody" herein refers to a genetically engineered non-human antibody that has an amino acid sequence modified to increase homology to the sequence of a human antibody. Generally, all or part of the CDRs of a humanized antibody is derived from a non-human antibody (donor antibody), and all or part of the non-CDRs (e.g., variable region FRs and/or constant regions) is derived from a human immunoglobulin (receptor antibody). The humanized antibody generally retains or partially retains the desired properties of the donor antibody, including, but not limited to, antigen specificity, affinity, reactivity, the ability to increase the activity of immune cells, the ability to enhance immune response, and the like.

The term "fully human antibody" herein refers to an antibody having variable regions in which both the FRs and CDRs are derived from human germline immunoglobulin sequences. Furthermore, if the antibody comprises constant regions, the constant regions are also derived from human germline immunoglobulin sequences. The fully human antibody herein may include amino acid residues that are not encoded by human germline immunoglobulin sequences (e.g., mutations introduced by random or site-specific mutagenesis *in vitro* or by somatic mutations *in vivo*)*.* However, "fully human antibody" herein is not intended to include antibodies in which CDR sequences derived from the germline of another mammalian species (e.g., mouse) have been grafted onto human framework sequences.

The term "naked antibody" herein refers to an antibody that is not linked, fused, or conjugated to another agent or molecule (e.g., a label or drug), peptide, or polypeptide. In specific embodiments, the naked antibody expressed by mammalian host cells can be glycosylated by the glycosylation machinery (e.g., glycosylases) of the host cells. In certain embodiments, the naked antibody is not glycosylated when expressed by a host cell that does not have its own glycosylation machinery (e.g., a glycosylase). In certain embodiments, naked antibodies are intact antibodies, while in other embodiments, naked antibodies are antigen-binding fragments of intact antibodies, e.g., Fab antibodies.

The term "conjugated antibody" herein refers to an antibody that may be associated with a pharmaceutically acceptable carrier or diluent, which may be a monoclonal antibody, a chimeric antibody, a humanized antibody, or a human antibody.

The term "diabody" herein refers to a bivalent bispecific antibody that can bind to different epitopes on the same or different antigens.

The term "minimal recognition unit of an antibody" herein refers to the minimal unit of an antibody that can recognize an antigen in an antigen-antibody binding reaction.

The term "nanobody" herein refers to a heavy chain antibody naturally lacking a light chain present in a camel, and the cloning of its variable region can give a single domain antibody only consisting of a heavy chain variable region (also called VHH (variable domain of heavy chain of heavy chain antibody)), which is the smallest functional antigen-binding fragment.

As used herein, the term "percent (%) sequence identity" refers to the percentage of identity between amino acid (or nucleotide) residues of a candidate sequence and amino acid (or nucleotide) residues of a reference sequence after aligning the sequences and introducing gaps, if necessary, to achieve maximum percent sequence identity (e.g., gaps may be introduced in one or both of the candidate sequence and the reference sequence for optimal alignment, and non-homologous sequences may be omitted for the purpose of comparison). Alignment may be carried out in a variety of ways well known to those skilled in the art for the purpose of determining percent sequence identity, for example, using publicly available computer software such as BLAST, ALIGN, or Megalign (DNASTAIi) software. Those skilled in the art can determine appropriate parameters for measuring alignment, including any algorithms required to achieve maximum alignment of the full length of the aligned sequences. For example, a reference sequence aligned for comparison with a candidate sequence may show that the candidate sequence exhibits 50% to 100% sequence identity over the full length of the candidate sequence or a selected portion of contiguous amino acid (or nucleotide) residues of the candidate sequence. The length of the candidate sequence aligned for the purpose of comparison may be, e.g., at least 30% (e.g., 30%, 40%, 50%, 60%, 70%, 80%, 90%, or 100%) of the length of the reference sequence. When a position in the candidate sequence is occupied by the same amino acid (or nucleotide) residue at the corresponding position in the reference sequence, then the molecules are identical at that position.

The term "conservative amino acid" herein generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
(1) acidic amino acids: Asp (D) and Glu (E);
(2) basic amino acids: Lys (K), Arg (R), and His (H);
(3) hydrophilic uncharged amino acids: Ser (S), Thr (T), Asn (N), and Gln (Q);
(4) aliphatic uncharged amino acids: Gly (G), Ala (A), Val (V), Leu (L), and Ile (I);
(5) non-polar uncharged amino acids: Cys (C), Met (M), and Pro (P); and
(6) aromatic amino acids: Phe (F), Tyr (Y), and Trp (W).

The term "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991, the content of which is incorporated herein by reference).

As used herein, the term "specifically binding" refers to a binding reaction that determines the presence of an antigen in a heterogeneous population of proteins and other biomolecules that are specifically recognized, e.g., by antibodies or antigen-binding fragments thereof. An antibody or an antigen-binding fragment thereof that specifically binds to an antigen will bind to the antigen with a KD of less than 100 nM. For example, an antibody or an antigen-binding fragment thereof that specifically binds to an antigen will bind to the antigen with a KD of up to 100 nM (e.g., between 1 pM and 100 nM). Antibodies or antigen-binding fragments thereof that do not exhibit specific binding to a particular antigen or epitope thereof will exhibit a KD of greater than 100 nM (e.g., greater than 500 nM, 1 µM, 100 µM, 500 µM, or 1 mM) for the particular antigen or epitope thereof. A variety of immunoassay methods may be used to select antibodies specifically immunoreactive with a particular protein or carbohydrate. For example, solid-phase ELISA immunoassays are routinely used to select antibodies specifically immunoreactive with a protein or carbohydrate. See Harlow & Lane, Antibodies, A Laboratory Manual, Cold Spring Harbor Press, New York (1988) and Harlow & Lane, Using Antibodies, A Laboratory Manual, Cold Spring Harbor Press, New York (1999), which describe immunoassay methods and conditions that can be used to determine specific immunoreactivity (which are incorporated herein by reference).

As used herein, the term "antibody conjugate" refers to a conjugate formed by an antibody molecule chemically bonded to an additional molecule directly or through a linker, e.g., an antibody-drug conjugate (ADC) in which the drug molecule is the additional molecule.

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a sugar (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. Herein, the term "nucleic acid molecule" encompasses deoxyribonucleic acid (DNA), including, e.g., complementary DNA (cDNA) and genomic DNA; ribonucleic acid (RNA), in particular messenger RNA (mRNA); the synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain naturally occurring or non-naturally occurring nucleotides. Examples of non-naturally occurring nucleotides include modified nucleotide bases having derived sugar or phosphate backbone bonding or chemically modified residues. The nucleic acid molecule also encompasses DNA and RNA molecules suitable for use as vectors for direct expression of the antibodies of the present disclosure *in vitro* and/or *in vivo,* e.g., in a host or patient. Such DNA (e.g., cDNA) or RNA (e.g., mRNA) vectors may be unmodified or modified. For example, mRNA may be chemically modified to enhance the stability of the RNA vector and/or the expression of the encoded molecule, so that the mRNA can be injected into a subject to produce antibodies *in vivo* (see, e.g., Stadler et al., Nature Medicine 2017, published online, June 12, 2017, doi: 10.1038/nm.4356 or EP 2 101 823 B1, the content of which is incorporated herein by reference).

As used herein, the term "vector" includes nucleic acid vectors, e.g., DNA vectors (e.g., plasmids), RNA vectors, viruses, or other suitable replicons (e.g., viral vectors). Various vectors have been developed for the delivery of polynucleotides encoding foreign proteins into prokaryotic or eukaryotic cells. The expression vector of the present disclosure contains polynucleotide sequences as well as additional sequence elements, e.g., for expressing proteins and/or integrating these polynucleotide sequences into the genome of mammalian cells. Some vectors that may be used to express the antibody and antibody fragment of the present disclosure include plasmids comprising regulatory sequences (e.g., promoter and enhancer regions) that direct gene transcription. Other useful vectors for expressing the antibody and antibody fragment contain polynucleotide sequences that enhance the rate of translation of these genes or improve the stability or nuclear export of mRNA produced by gene transcription. These sequence elements include, for example, 5' and 3' untranslated regions, internal ribosome entry sites (IRESs), and polyadenylation signal sites, so as to direct the effective transcription of the gene carried on the expression vector. The expression vector of the present disclosure may also contain a polynucleotide encoding a marker for selecting cells comprising such a vector. Examples of suitable markers include genes encoding resistance to antibiotics (e.g., ampicillin, chloramphenicol, kanamycin, or nourseothricin).

The term "host cell" herein refers to a cell into which an exogenous nucleic acid has been introduced, including the progeny of such a cell. Host cells include "transformants" and "transformed cells", which include primary transformed cells and progenies derived therefrom, regardless of the number of passages. Progenies may not be exactly the same as parent cells in terms of nucleic acid content, and may contain mutations. Mutant progenies having the same function or biological activity that are screened or selected from the primary transformed cells are included herein.

The term "pharmaceutical composition" herein refers to a formulation that exists in a form allowing the biological activity of the active ingredient contained therein to be effective and does not contain additional ingredients having unacceptable toxicity to a subject to which the pharmaceutical composition is administered.

As used herein, the terms "subject" and "patient" refer to an organism that receives a treatment for a particular disease or disorder (e.g., a cancer or an infectious disease) as described herein. Examples of the subject and the patient include mammals, such as human, primate, pig, goat, rabbit, hamster, cat, dog, guinea pig, members of the bovine family (such as cattle, bison, buffalo, elk, yak, etc.), sheep, horse, etc., that receive a treatment for a disease or disorder (e.g., a cell proliferative disorder, such as a cancer or an infectious disease).

As used herein, the term "treatment" refers to surgical or therapeutic treatment for the purpose of preventing or slowing (reducing) the progression of an undesirable physiological or pathological change, such as a cell proliferative disorder (e.g., a cancer or an infectious disease), in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

The term "effective amount" herein refers to an amount of a therapeutic agent that is effective to prevent or alleviate symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "appropriate condition" herein refers to conditions suitable for culturing various host cells, including eukaryotic cells and prokaryotic cells.

The term "tumor" herein refers to all neoplastic cell growth and proliferation, whether malignant or benign, and all pre-cancerous and cancerous cells and tissues. The terms "cancer" and "tumor" are not mutually exclusive when referred to herein.

The term "antineoplastic agent" herein refers to an anti-tumor drug, which is a class of drugs for treating tumor diseases, such as a chemotherapeutic drug and a biological formulation.

The term "EC50" herein refers to the half maximum effective concentration, which includes the antibody concentration that induces a halfway response between the baseline and maximum after a specified exposure time. EC50 essentially represents the antibody concentration at which 50% of the maximal effect is observed, and can be measured by methods known in the art.

### BRIEF DESCRIPTION OF THE DRAWINGS

Unless otherwise defined herein, scientific and technical terms used in correlation with the present disclosure shall have the meanings that are commonly understood by those of ordinary skill in the art.
FIGs. 1-2 show the binding activity of hCDH6-1 humanized antibody to human CDH6 protein.
FIGs. 3-4 show the binding activity of hCDH6-1 humanized antibody to OVCAR3 cells.
FIGs. 5-6 show the binding activity of hCDH6-6 humanized antibody to human CDH6 protein.
FIGs. 7-8 show the binding activity of hCDH6-6 humanized antibody to OVCAR3 cells.
FIGs. 9-11 show the binding activity of hCDH6-14 humanized antibody to human CDH6 protein.
FIGs. 12-13 show the binding activity of hCDH6-14 humanized antibody to PA-1 cells.

### DETAILED DESCRIPTION

The present disclosure will be further described with reference to specific examples, and the advantages and features of the present disclosure will become more apparent with the description. Experimental procedures without specified conditions in the examples are conducted according to conventional conditions or conditions recommended by the manufacturers. Reagents or instruments without specified manufacturers used herein are conventional products that are commercially available.

The examples are exemplary only and do not limit the scope of the present disclosure in any way. It will be understood by those skilled in the art that various changes or substitutions in form and details may be made to the technical solutions of the present disclosure without departing from the spirit and scope of the present disclosure, and that these changes and substitutions shall fall within the protection scope of the present disclosure.

### Example 1. Preparation of Anti-Human CDH6 Murine Monoclonal Antibodies

### 1.1 Immunization of animals

### 1.1.1. Protein immunization

5 SJL mice (Nos. #2401-2405) or 5 MRL/lpr mice (Nos. #2136-2140) (purchased from Shanghai SLAC Laboratory Animal Co., Ltd.) aged 6-8 weeks were used for immunization, and the mice were housed under SPF conditions. For the primary immunization, an Fc-tagged human CDH6 extracellular region protein (hCDH6 ECD-hFc, conventionally synthesized; the hCDH6 ECD sequence was derived from Uniprot# P55285 (19-615), set forth in SEQ ID NO: 1 below, conventionally synthesized) was mixed and emulsified with Titer max (purchased from Sigma, Cat. No. T2684) and oligonucleotide CPG (ODN 1826, synthesized from Sangon Biotech (Shanghai)), and then injected into the foot pad and back. The hCDH6 ECD-hFc protein was mixed with Imject Alum (purchased from Thermo fisher, Cat. No. 77161) and CPG, and then injected into the abdominal cavity, and 50 µg of antigen was injected into each mouse. For the first booster immunization, the hCDH6 ECD-hFc protein, Imject Alum, and CPG were mixed well and then injected into the foot pad and back. For the second booster immunization, the hCDH6 ECD-hFc protein, Titer max, and CPG were mixed and emulsified, and then injected into the back. The subsequent booster immunizations were alternately performed as the first and second booster immunizations. Each mouse was injected with 25 µg of antigen each time, with an interval of 7 days between each immunization. Blood was collected from the mice on day 5 after the second and fourth booster immunizations, and serum was separated. The titer of specific antibodies in the serum was determined by an enzyme-linked immunosorbent assay (ELISA).
hCDH6 ECD, SEQ ID NO: 1 (Uniprot# P55285 (19-615))

TLSTPLSKRTSGFPAKKRALELSGNSKNELNRSKRSWMWNQFFLLEEYTGSDYQYVGKLH
SDQDRGDGSLKYILSGDGAGDLFIINENTGDIQATKRLDREEKPVYILRAQAINRRTGRPVEP
ESEFIIKIHDINDNEPIFTKEVYTATVPEMSDVGTFVVQVTATDADDPTYGNSAKVVYSILQG
QPYFSVESETGIIKTALLNMDRENREQYQVVIQAKDMGGQMGGLSGTTTVNITLTDVNDNP
PRFPQSTYQFKTPESSPPGTPIGRIKASDADVGENAEIEYSITDGEGLDMFDVITDQETQEGII
TVKKLLDFEKKKVYTLKVEASNPYVEPRFLYLGPFKDSATVRIVVEDVDEPPVFSKLAYILQ
IREDAQINTTIGSVTAQDPDAARNPVKYSVDRHTDMDRIFNIDSGNGSIFTSKLLDRETLLW
HNITVIATEINNPKQSSRVPLYIKVLDVNDNAPEFAEFYETFVCEKAKADQLIQTLHAVDKD
DPYSGHQFSFSLAPEAASGSNFTIQDNKDNTAGILTRKNGYNRHEMSTYLLPVVISDNDYPV
QSSTGTVTVRVCACDHHGNMQSCHAEALIHPTGLSTGA

The ELISA assay method was as follows: A his-tagged human CDH6 extracellular region protein (hCDH6 ECD-his, conventionally synthesized; the hCDH6 ECD sequence was derived from Uniprot# P55285 (19-615), set forth in SEQ ID NO: 1) was diluted with PBS to a final concentration of 1 µg/mL and added to a 96-well ELISA plate at 100 µL/well, and the plate was incubated overnight at 4 °C. The next day, the plate was washed 3 times with a plate washing solution (PBS + 0.01% (v/v) Tween 20), and a blocking buffer (PBS + 0.01% (v/v) Tween 20 + 2% (w/w) BSA) was added for blocking at room temperature for 2 h. The blocking buffer was discarded, and the plate was washed 3 times with a plate washing solution. Mouse serum was diluted with 1% BSA + PBS and added to the 96-well ELISA plate at 50 µL/well. After incubation at 37 °C for 2 h, the plate was washed 3 times with a plate washing solution. A horseradish peroxidase-labeled secondary antibody (Peroxidase AffiniPure Goat Anti-Mouse IgG (H+L), purchased from Jackson Immuno, Cat. No. 115-035-003) diluted in a 1:5000 ratio was added at 50 µL/well. The plate was incubated at 37 °C for 1 h and then washed 3 times with a plate washing solution. A TMB substrate was added at 50 µL/well and incubated at room temperature for 10 min, then a stop solution (1.0 N HCl) was added at 50 µL/well. OD450 nm values were read using a microplate reader (Biotek).

The ELISA experimental results are shown in Table 1. After 5 immunizations, the serum of the immunized mice had relatively high titers of binding to human CDH6 protein, showing antigen-antibody reactions. TB2 refers to the serum of mice on day 5 after 5 immunizations (primary immunization + 4 booster immunizations), and the data in the table are OD450 nm values.

**Table 1. Serum titer of mice immunized with hCDH6 ECD-hFc protein assayed by ELISA**

| Mouse strain | MRL/lpr | | | | | SJL | | | | |
|---|---|---|---|---|---|---|---|---|---|---|
| Serum dilution factor | 2136 TB2 | 2137 TB2 | 2138 TB2 | 2139 TB2 | 2140 TB2 | 2401 TB2 | 2402 TB2 | 2403 TB2 | 2404 TB2 | 2405 TB2 |
| 1:0.3k | 1.15 | 1.14 | 1.10 | 1.08 | 1.07 | 1.05 | 1.09 | 1.19 | 1.17 | 1.12 |
| 1:0.9k | 1.19 | 1.14 | 1.25 | 1.07 | 1.16 | 1.06 | 1.13 | 1.14 | 1.15 | 1.07 |
| 1:2.7k | 1.08 | 1.18 | 1.19 | 1.12 | 1.11 | 1.22 | 1.12 | 1.15 | 1.11 | 1.11 |
| 1:8.1k | 1.09 | 1.19 | 1.17 | 1.03 | 1.07 | 1.23 | 1.24 | 1.15 | 1.10 | 1.05 |
| 1:24.3k | 1.06 | 1.29 | 1.26 | 1.10 | 1.17 | 1.39 | 1.22 | 1.19 | 1.23 | 1.11 |
| 1:72.9k | 1.03 | 1.28 | 1.30 | 1.13 | 1.17 | 0.87 | 1.37 | 1.43 | 1.28 | 1.25 |
| 1:218.7k | 0.99 | 1.06 | 1.24 | 1.36 | 1.20 | 0.40 | 1.02 | 1.04 | 1.07 | 0.87 |
| 1:656.1k | 0.52 | 0.52 | 0.62 | 0.80 | 0.57 | 0.19 | 0.53 | 0.47 | 0.52 | 0.40 |
| 1:1968.3k | 0.26 | 0.26 | 0.30 | 0.37 | 0.26 | 0.12 | 0.24 | 0.23 | 0.23 | 0.19 |
| 1:5904.9k | 0.14 | 0.13 | 0.16 | 0.19 | 0.12 | 0.08 | 0.13 | 0.13 | 0.11 | 0.12 |
| 1:17714.7k | 0.11 | 0.09 | 0.10 | 0.10 | 0.10 | 0.07 | 0.09 | 0.08 | 0.08 | 0.08 |
| 1:53144.1k | 0.10 | 0.09 | 0.08 | 0.08 | 0.08 | 0.07 | 0.07 | 0.07 | 0.07 | 0.07 |
| Blank control | 0.07 | | | | | | | | | |

### 1.1.2. Cell and protein immunization

3 SJL mice (Nos. #2453, 2454, and 2456) or 5 MRL/lpr mice (Nos. #2448-2452) (purchased from Shanghai SLAC Laboratory Animal Co., Ltd.) aged 6-8 weeks were used for immunization, and the mice were housed under SPF conditions. For primary immunization, HEK293T cells overexpressing human CDH6 protein (HEK293T-hCDH6, conventionally constructed; HEK293T cells were purchased from Cell Bank of Chinese Academy of Sciences, Cat. No. SCSP-502) were mixed with Titer max (purchased from Sigma, Cat. No. T2684) and then injected intraperitoneally at 5×10⁶ cells per mouse. For the first booster immunization, HEK293T-hCDH6 cells and CPG were mixed well and then injected intraperitoneally; for the second booster immunization, HEK293T-hCDH6 cells and Titer max were mixed and then injected intraperitoneally. The subsequent booster immunizations were alternately performed as the first and second booster immunizations. Each mouse was injected with 5×10⁶ cells each time, with an interval of 7 days between each immunization. Blood was collected from the mice on day 5 after the second and fourth booster immunizations, and serum was separated. The titer of specific antibodies in the serum was determined by ELISA. According to the titer of mice, mice were immunized with hCDH6 ECD-hFc protein (the protein source was the same as that in 1.1.1) after 5 cell immunizations (primary immunization + 4 booster immunizations).

The titer of specific antibodies in the serum was determined by an enzyme-linked immunosorbent assay (ELISA). The ELISA experimental results are shown in Table 2. After 5 cell immunizations and 3 protein immunizations, the serum of the immunized mice had relatively high titers of binding to human CDH6 protein, showing antigen-antibody reactions. TB4 refers to the serum of mice on day 5 after 8 immunizations, and the data in the table are OD450 nm values.

**Table 2. Serum titer of mice immunized with HEK293T-hCDH6 cells and hCDH6 ECD-hFc protein assayed by ELISA**

| Mouse strain | MRL/lpr | | | | | SJL | | |
|---|---|---|---|---|---|---|---|---|
| Serum dilution factor | 2448 TB4 | 2449 TB4 | 2450 TB4 | 2451 TB4 | 2452 TB4 | 2453 TB4 | 2454 TB4 | 2456 TB4 |
| 1:0.1k | 2.75 | 2.63 | 3.02 | 2.85 | 2.84 | 2.81 | 2.82 | 3.00 |
| 1:0.3k | 2.51 | 2.71 | 2.89 | 2.52 | 2.52 | 2.89 | 2.88 | 2.78 |
| 1:0.9k | 2.34 | 2.55 | 2.47 | 2.41 | 2.41 | 2.57 | 2.70 | 2.61 |
| 1:2.7k | 1.67 | 2.26 | 2.15 | 2.36 | 2.15 | 2.44 | 2.49 | 2.72 |
| 1:8.1k | 0.96 | 1.66 | 1.55 | 1.57 | 1.61 | 1.97 | 1.80 | 2.20 |
| 1:24.3k | 0.41 | 1.02 | 0.82 | 0.86 | 0.84 | 1.08 | 1.01 | 1.77 |
| 1:72.9k | 0.20 | 0.51 | 0.42 | 0.54 | 0.64 | 0.50 | 0.51 | 0.90 |
| 1:218.7k | 0.12 | 0.27 | 0.26 | 0.26 | 0.25 | 0.24 | 0.21 | 0.40 |
| 1:656.1k | 0.06 | 0.10 | 0.09 | 0.14 | 0.13 | 0.11 | 0.10 | 0.18 |
| 1:1968.3k | 0.05 | 0.08 | 0.21 | 0.41 | 0.08 | 0.07 | 0.06 | 0.14 |
| 1:5904.9k | 0.04 | 0.05 | 0.05 | 0.07 | 0.06 | 0.05 | 0.05 | 0.06 |
| Blank control | 0.04 | | | | | | | |

### 1.2 Hybridoma preparation

The mice with higher titer were selected and injected with 50 µg of hCDH6 ECD-hFc protein (the protein source was the same as that in 1.1.1) in the abdominal cavity, plantar part and back. The mice were sacrificed 3 days later, and spleen cells and lymphocytes were collected. After centrifugation at 1500 rpm/min, the supernatant was discarded, and ACK lysis buffer (purchased from Gibco, Cat. No. A1049201) was added to the cells to lyse red blood cells mixed in the cells to obtain a cell suspension. The cells were washed 3 times with a DMEM basal medium (purchased from Gibco, Cat. No. 10569044) at 1500 rpm/min, then mixed with mouse myeloma cells SP2/0 (purchased from ATCC, Cat. No. CRL-1581) at a viable cell count of 2:1, and the resulting mixture was subjected to cell fusion by an electrofusion method (instrument: BTX 2001+). The fused cells were diluted in a DMEM medium containing 20% (w/w) fetal bovine serum (purchased from ExCell Bio, Cat. No. FND500), 1× HAT (purchased from Sigma, Cat. No. H0262-10VL), bovine insulin (purchased from Yeason, Cat. No. 40107ES25), and NEAA (purchased from Gibco, Cat. No. 11140050), and then added to a 96-well cell culture plate at 5×10⁴ cells/200 µL/well. The plate was put into an incubator with 5% (v/v) CO₂ at 37 °C for culturing.

After 7 days, the fusion plate supernatants were screened by ELISA to determine the binding activity to human CDH6 protein; the binding activity of the positive clone supernatant to monkey CDH6 protein, murine CDH6 protein, and cells overexpressing human CDH6 was determined by ELISA. According to the screening results, eligible positive clones were selected and subcloned using a semi-solid medium (purchased from Stemcell, Cat. No. 03810). After 7 days, the grown clones were picked one by one into a 96-well culture plate and expanded in a DMEM medium containing 10% (w/w) fetal bovine serum and 1× HT (purchased from Sigma, Cat. No. H0137-10VL). After 1 day, a preliminary screening was performed using an ELISA method, and single clones having binding activity to human CDH6 protein were selected and expanded into a 24-well plate for further culture. After 3 days, the culture supernatant was further detected to evaluate the binding activity to the monkey CDH6 protein and the murine CDH6 protein and the endocytic activity on cells overexpressing human CDH6. According to the detection results of the samples in the 24-well plate, the optimal clones were selected and expanded in a DMEM medium containing 10% (w/w) FBS at 37 °C with 5% (v/v) CO₂. The cells were cryopreserved in liquid nitrogen to obtain the optimal hybridoma cells, which could be used for subsequent antibody production and purification.

### Example 2. Preparation and Identification of Chimeric Antibodies

### 2.1. Preparation of chimeric antibodies

The hybridoma clones with good activity in Example 1 were selected for sequencing. The sequences of the positive control anti-human CDH6 antibodies DS-H01L02 and NOV0712 were derived from the patent WO2018212136A1. The corresponding sequence information is shown in Table 3 and Table 4 below, wherein Table 3 shows the VH and VL sequences of the murine monoclonal antibodies, and Table 4 shows the CDRs of the murine monoclonal antibody molecules (KABAT analysis).

**Table 3. VH and VL sequences of murine monoclonal antibodies and positive control antibodies, and constant region sequences of chimeric antibodies**

| Antibody | Sequence No. | Amino acid sequence |
|---|---|---|
| CDH6-1-VH | SEQ ID NO: 2 | QVQLQQPGAELVRPGSSVKLSCKASGYTFTNYWMHWVKQRPIE GLEWIGDIDPSHSETHYTQKFKDKATLTVDKSSSTAYMQLSSLTS EDSAVYYCARGDLGRFDYWGQGTTLTVSS |
| CDH6-1-VL | SEQ ID NO: 3 | DIVLTQSPASLAVSLGQRATISCRASESVSIHGTHLMHWYQQKSG QPPKLLIYAASNLESGVPARFSGSGSETDFTFNIHPVEEEDAATYF CQQSFEDPWTFGGGTKLEIK |
| CDH6-2-VH | SEQ ID NO: 4 | EVQLQQSGPELVKPGASVRISCKASGYTFTDYYMNWVKQSHGK SLEWIGDIDPNNANTTYNQKFKGKATLTVDKSSSTAYMELRSLTS EDSAVYSCASFGYDVEWFGYWGQGTLVTVSA |
| CDH6-2-VL | SEQ ID NO: 5 | QIVLTQSPALMSASPGEKVTMTCSASSSVSYMYWYQQKPRSSPK PWIYLTSNLASGVPARFSGSGSGTSYSLTISSMEAEDAATYYCQQ WSSNPFTFGSGTKLEIK |
| CDH6-3-VH | SEQ ID NO: 6 | QVQLQQPGAELVKPGASVKLSCKASGYTFTSYWIHWVKQRPGQ GLEWIGEINPSNGVTNCNENFKSKATLTVDKSSSTAYMQLSSLTS EDSAVYYCTIPNLLWWYFDVWGAGTTVTVSS |
| CDH6-3-VL | SEQ ID NO: 7 | DIQMTQSPASLSVSVGETVTITCRASENIYSNLAWYQQKQGKSPQ LLVYEATNLADGVPSRFSGSGSGTQYSLKINSLQSEDFGSYYCQH FWGTLWTFGGGTKLEIK |
| CDH6-4-VH | SEQ ID NO: 8 | QVQLQQSGPELVKPGASVKISCKASGYAFSNFWMNWVKQRPGK GLEWIGWIYPGDGDTNYNGKFKDKATLTADKSSSTAYIQLSSLTS EDSAVYFCARLYDAWGFFDYWGQGTTLTVSS |
| CDH6-4-VL | SEQ ID NO: 9 | QIVLTQSPAIMSASPGQKVTITCSASSSVNYMHWYQQKLGSSPKL WIYDKSKLAPGVPARFSGSGSGTSYSLTISSMEAEDAASYFCHQ WSSYPPMTFGGGTKLEIK |
| CDH6-5-VH | SEQ ID NO: 10 | QVQLQQSGAELVRPGTSVKMSCKAAEYTFTNYWIGWVKQRPG HGLEWIGDLYPGGDHANYNEKFEGKATLTADTSSSTAYMQLSSL TSEDSAIYYCARGMLTTGFDSWGQGTTLTVSS |
| CDH6-5-VL | SEQ ID NO: 11 | DIVMSQSPSSLAVSAGEKVTMSCKSSQSLFNSRTRKNYLAWYQQ KPGQSPKLLIYWASTRESGVPDRFTGSGSGTDFTLTISSVQAEDLA VYYCKQSYYLRTFGGGTKLEIK |
| CDH6-6-VH | SEQ ID NO: 12 | EVQLQQSGPELVKPGDSVKMSCKASGYTFTDYYMAWVRQSHR KSLEWIGYIFPNSGGTDYSQKFKGKATLTVDKSSSTVYMELHSLT SEDSAVYYCARELGADYGDYYAMDHWGQGTSVSVSS |
| CDH6-6-VL | SEQ ID NO: 13 | DIQMTQSPASLSASVGETVTITCRASENIDSYLAWYQQKQGKSPQ LLVYSATLLADGVPSRFSGSGSGTQYSLKINSLKSEDVARYYCQH YYRTPPTFGSGTKLEIK |
| CDH6-7-VH | SEQ ID NO: 14 | EVKLVESGGGLVKPGGSLKVSCAASGFTFSDHGMAWVRQAPGK GPEWVAFISNLAYSIYYADTVTGRFTISRENAKNTLYLEMSSLRSE DTAMYYCARVYVKWYFDVWGAGTTVTVSS |
| CDH6-7-VL | SEQ ID NO: 15 | DIQMTQSPASLSASVGETVTITCRASENIDSYLAWYQQKQGKSPQ LLVFAATLLADGVPSRFSGSGSGTQYSLKINSLQSEDVARYYCQH YYSTPLTFGAGTKLELK |
| CDH6-8-VH | SEQ ID NO: 16 | EVQLQQSGPELVKPGDSVKMSCKASGYTFTDYYMAWIKQNHG KSLEWIGYIYPNNGGTDYNQKFRGKATLTVDKSSSAAYMELHSL TSEDSAVYYCAREIGTLYGDYYAMDYWGQGTSVTVSS |
| CDH6-8-VL | SEQ ID NO: 17 | DIQMTQSPASLSASVGETVTITCRASENIDSYLAWYQQKQGKSPQ LLVYTATLLADGVPSRFSGSGSGTQYSLKINSLQSEDVARYYCQH YYSTPPTFGSGTKLEIK |
| CDH6-9-VH | SEQ ID NO: 18 | QVQLQQPGAELVKPGASVKLSCKASGYTFTGYWMHWVRQRPG QGLEWIGEINPNNGGTNCNEQFKNKATLTVDKSSSTAYMQLSSLT SEDSAVFYCTISFGNYLFFDVWGAGTTVTVSS |
| CDH6-9-VL | SEQ ID NO: 19 | DIQMTQSPASLAASVGETITITCQASENIYFSLAWYQQKQGKSPQ LLIYNANTLEDGVPSRFVGSGSGTQYSMKINSIQPEDTATYFCDQ TYDFPWTFGGGTKLEIK |
| CDH6-10-VH | SEQ ID NO: 20 | QVQLQQPGAELVKPGTSVKMSCKASGYTFTSDWIAWVKQRPGQ GLEWIGDIYPGSGRTHNNEKFNFKATLTVDTSSNTAYMQLSSLTS EDSAVYFCARPMLGRFDYWGQGTTLTVSS |
| CDH6-10-VL | SEQ ID NO: 21 | DIVLTQSPASLAVSLGQRATISCRASESVSIHGTHLMHWYQQKPG QPPKLLIYAASNLESGVPARFSGSGSETDFTLNIHPVEEEDAATYF CQQSFEDPFTFGSGTKLEIK |
| CDH6-11-VH | SEQ ID NO: 22 | QIQLQQSGPELVKPGASVRISCKASGYTFTDYYITWVKLRPGQGL EWIGWIYPGSVNIKYNEKFKGKATLTVDTSSSTAYMQLNSLTSED SAVYFCARGGYSWFPYWGQGTLVTVSA |
| CDH6-11-VL | SEQ ID NO: 23 | NIVMTQSPKSMSMSVGERVTLSCKASENVHTYVSWYQQKPEQS PKLLFYGASTRNTGVPDRFTGSGSATDFTLTISSVQAEDLADYYC GQSYSYPYTFGSGTKLEIK |
| CDH6-12-VH | SEQ ID NO: 24 | EVNLVESGGGLVQPGDSLTLSCSASGFTFTDYYMSWVRQFPGKA LEWLALIRNKANSYTTEYSASVKGRFTISRDNSQSILYLQMNALR AEDSATYYCARDSYYSHDGFAYWGQGTLVTVSA |
| CDH6-12-VL | SEQ ID NO: 25 | DIVLTQSPASLAVSLGQRATISCRASKSVSTSSYTYMHWYQQKPG QPPKLLIKFASYLESGVPARFSGSGSGTDFTLNIHPVEEEDAATYY CQHSREFPFTFGTGTKLEIK |
| CDH6-13-VH | SEQ ID NO: 26 | DVQLQESGPGLVKPSQSLSLTCSVTGYSITKDSYWNWIRQFPGSK LEWMGFISYDGSRHYNPSLKNRISITRDTSKNQFFLQLNSVTTED TATYYCARRDYADYWGQGTTLTVSS |
| CDH6-13-VL | SEQ ID NO: 27 | QIVLTQSPALMSAFPGEKVTMTCSATSSVSYIYWYQQKPRSSPKP WIYLTSNLASGVPARFSGSGSGTSYSLTINSLEAEDAATYYCQQW SSIPYTFGGGTKLEIK |
| CDH6-14-VH | SEQ ID NO: 28 | QIQLQQSGPELVKPGTSVKISCKASGYTFTDNYINWVRQRPGQG LEWIGWIYPGTGNSYYNEKFKGKATLTVDTSSSTAYMQFSSLTSE DSAVYLCASSYGDSFDYWGQGTTLTVSS |
| CDH6-14-VL | SEQ ID NO: 29 | DIQMTQTTSSLSASLGNRVTISCRASQDIRNYLNWYQQKPDGTF KLLISYTSRLHSGVPSRFSGSGSGTDYSLTVSNLEPEDIATYYCQQ YSQLPWTFGGGSRLEIK |
| DS-H01L 02-VH | SEQ ID NO: 30 | EVQLVQSGAEVKKPGASVKVSCKASGYTFTRNFMHWVRQAPG QGLEWMGWIYPGDGETEYAQKFQGRVTITADTSTSTAYMELSSL RSEDTAVYYCARGVYGGFAGGYFDFWGQGTLVTVSS |
| DS-H01L 02-VL | SEQ ID NO: 31 | DIQMTQSPSSLSASVGDRVTITCKASQNIYKNLAWYQQKPGKAP KLLIYDANTLQTGVPSRFSGSGSGSDFTLTISSLQPEDFATYFCQQ YYSGWAFGQGTKVEIK |
| NOV0712 -VH | SEQ ID NO: 32 | QVQLLESGGGLVQPGGSLRLSCAASGFTFSSHGMHWVRQAPGK GLEWVSVISGSGSNTGYADSVKGRFTISRDNSKNTLYLQMNSLR AEDTAVYYCARQWGSYAFDSWGQGTLVTVSS |
| NOV0712 -VL | SEQ ID NO: 33 | DIQMTQSPSSLSASVGDRVTITCRASQSISSYLNWYQQKPGKAPK LLIYAVSTLQSGVPSRFSGSGSGTDFTLTISSLQPEDFATYYCQQSG TFPPTTFGQGTKVEIK |
| CH1-Fc | SEQ ID NO: 34 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPELLGGPSVFLFPPKPKDTLMI SRTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSREEMTKNQVSLTCLVKGFYPSDIAVEWESNG QPENNYKTTPPVLDSDGSFFLYSKLTVDKSRWQQGNVFSCSVMH EALHNHYTQKSLSLSPGK |
| CL | SEQ ID NO: 35 | |

**Table 4. CDRs of murine monoclonal antibody molecules and positive control antibodies (KABAT analysis)**

| Antibody | CDR1 | Sequence No. | CDR2 | Sequence No. | CDR3 | Sequence No. |
|---|---|---|---|---|---|---|
| CDH6-1-VH | NYWMH | SEQ ID NO: 36 | DIDPSHSETHYTQKF KD | SEQ ID NO: 37 | GDLGRFDY | SEQ ID NO: 38 |
| CDH6-1-VL | RASESVSIHGTH LMH | SEQ ID NO: 39 | AASNLES | SEQ ID NO: 40 | QQSFEDPWT | SEQ ID NO: 41 |
| CDH6-2-VH | DYYMN | SEQ ID NO: 42 | DIDPNNANTTYNQKF KG | SEQ ID NO: 43 | FGYDVEWFGY | SEQ ID NO: 44 |
| CDH6-2-VL | SASSSVSYMY | SEQ ID NO: 45 | LTSNLAS | SEQ ID NO: 46 | QQWSSNPFT | SEQ ID NO: 47 |
| CDH6-3-VH | SYWIH | SEQ ID NO: 48 | EINPSNGVTNCNENF KS | SEQ ID NO: 49 | PNLLWWYFDV | SEQ ID NO: 50 |
| CDH6-3-VL | RASENIYSNLA | SEQ ID NO: 51 | EATNLAD | SEQ ID NO: 52 | QHFWGTLWT | SEQ ID NO: 53 |
| CDH6-4-VH | NFWMN | SEQ ID NO: 54 | WIYPGDGDTNYNGK FKD | SEQ ID NO: 55 | LYDAWGFFDY | SEQ ID NO: 56 |
| CDH6-4-VL | SASSSVNYMH | SEQ ID NO: 57 | DKSKLAP | SEQ ID NO: 58 | HQWSSYPPMT | SEQ ID NO: 59 |
| CDH6-5-VH | NYWIG | SEQ ID NO: 60 | DLYPGGDHANYNEK FEG | SEQ ID NO: 61 | GMLTTGFDS | SEQ ID NO: 62 |
| CDH6-5-VL | KSSQSLFNSRTR KNYLA | SEQ ID NO: 63 | WASTRES | SEQ ID NO: 64 | KQSYYLRT | SEQ ID NO: 65 |
| CDH6-6-VH | DYYMA | SEQ ID NO: 66 | YIFPNSGGTDYSQKF KG | SEQ ID NO: 67 | ELGADYGDYYAM DH | SEQ ID NO: 68 |
| CDH6-6-VL | RASENIDSYLA | SEQ ID NO: 69 | SATLLAD | SEQ ID NO: 70 | QHYYRTPPT | SEQ ID NO: 71 |
| CDH6-7-VH | DHGMA | SEQ ID NO: 72 | FISNLAYSIYYADTVT G | SEQ ID NO: 73 | VYVKWYFDV | SEQ ID NO: 74 |
| CDH6-7-VL | RASENIDSYLA | SEQ ID NO: 75 | AATLLAD | SEQ ID NO: 76 | QHYYSTPLT | SEQ ID NO: 77 |
| CDH6-8-VH | DYYMA | SEQ ID NO: 78 | YIYPNNGGTDYNQK FRG | SEQ ID NO: 79 | EIGTLYGDYYAMD Y | SEQ ID NO: 80 |
| CDH6-8-VL | RASENIDSYLA | SEQ ID NO: 81 | TATLLAD | SEQ ID NO: 82 | QHYYSTPPT | SEQ ID NO: 83 |
| CDH6-9-VH | GYWMH | SEQ ID NO: 84 | EINPNNGGTNCNEQF KN | SEQ ID NO: 85 | SFGNYLFFDV | SEQ ID NO: 86 |
| CDH6-9-VL | QASENIYFSLA | SEQ ID NO: 87 | NANTLED | SEQ ID NO: 88 | DQTYDFPWT | SEQ ID NO: 89 |
| CDH6-10-VH | SDWIA | SEQ ID NO: 90 | DIYPGSGRTHNNEKF NF | SEQ ID NO: 91 | PMLGRFDY | SEQ ID NO: 92 |
| CDH6-10-VL | RASESVSIHGTH LMH | SEQ ID NO: 93 | AASNLES | SEQ ID NO: 94 | QQSFEDPFT | SEQ ID NO: 95 |
| CDH6-11-VH | DYYIT | SEQ ID NO: 96 | WIYPGSVNIKYNEKF KG | SEQ ID NO: 97 | GGYSWFPY | SEQ ID NO: 98 |
| CDH6-11-VL | KASENVHTYVS | SEQ ID NO: 99 | GASTRNT | SEQ ID NO: 100 | GQSYSYPYT | SEQ ID NO: 101 |
| CDH6-12-VH | DYYMS | SEQ ID NO: 102 | LIRNKANSYTTEYSA SVKG | SEQ ID NO: 103 | DSYYSHDGFAY | SEQ ID NO: 104 |
| CDH6-12-VL | RASKSVSTSSY TYMH | SEQ ID NO: 105 | FASYLES | SEQ ID NO: 106 | QHSREFPFT | SEQ ID NO: 107 |
| CDH6-13-VH | KDSYWN | SEQ ID NO: 108 | FISYDGSRHYNPSLK N | SEQ ID NO: 109 | RDYADY | SEQ ID NO: 110 |
| CDH6-13-VL | SATSSVSYIY | SEQ ID NO: 111 | LTSNLAS | SEQ ID NO: 112 | QQWSSIPYT | SEQ ID NO: 113 |
| CDH6-14-VH | DNYIN | SEQ ID NO: 114 | WIYPGTGNSYYNEK FKG | SEQ ID NO: 115 | SYGDSFDY | SEQ ID NO: 116 |
| CDH6-14-VL | RASQDIRNYLN | SEQ ID NO: 117 | YTSRLHS | SEQ ID NO: 118 | QQYSQLPWT | SEQ ID NO: 119 |
| DS-H01L02-VH | RNFMH | SEQ ID NO: 120 | WIYPGDGETEYAQKF QG | SEQ ID NO: 121 | GVYGGFAGGYFDF | SEQ ID NO: 122 |
| DS-H01L02-VL | KASQNIYKNLA | SEQ ID NO: 123 | DANTLQT | SEQ ID NO: 124 | QQYYSGWA | SEQ ID NO: 125 |
| NOV0712-VH | SHGMH | SEQ ID NO: 126 | VISGSGSNTGYADSV KG | SEQ ID NO: 127 | QWGSYAFDS | SEQ ID NO: 128 |
| NOV0712-VL | RASQSISSYLN | SEQ ID NO: 129 | AVSTLQS | SEQ ID NO: 130 | QQSGTFPPTT | SEQ ID NO: 131 |

According to the above sequences, heavy chain (HC) and light chain (LC) plasmids were constructed (the nucleic acid sequences encoding the VH and VL of the antibodies were recombined into an expression vector carrying a signal peptide (SEQ ID NO: 132, MGWSWILLFLLSVTAGVHS) and a heavy chain constant region/light chain constant region sequence to obtain recombinant plasmids expressing VH-CH1-Fc/VL-CL). The corresponding heavy chain and light chain plasmids of the antibody and the transfection reagent PEI (purchased from Polysciences, Cat. No. 24765-1) were added to OPTI-ME M (purchased from Gibco, Cat. No. 11058021). The mixture was well mixed and let stand for 15 min. Expi293 cells (purchased from Thermofisher, Cat. No. A14527) were added, and the system was incubated on a shaker at 37 °C in 5% CO₂ at 120 rpm. On day 2 after the transfection, OPM-293 ProFeed (purchased from Shanghai OPM Biosciences Co., Ltd., Cat. No. F081918-001) and glucose (purchased from Sigma, Cat. No. G7528) were added. On day 6 after transfection, the cell supernatant was collected and purified using Protein A (purchased from GE, Cat. No. 28985254), and the eluted sample was dialyzed into PBS buffer (pH 7.4) to obtain the chimeric antibody and the positive control antibody.

### 2.2. Assay on binding of chimeric antibodies to human CDH6, monkey CDH6, and mouse CDH6 proteins by ELISA

Human CDH6 protein (hCDH6 ECD-his, conventionally synthesized), monkey CDH6 protein (cynoCDH6 ECD-his, conventionally synthesized; the cynoCDH6 ECD sequence was derived from Uniprot# A0A2K5TW62 (19-615)), and murine CDH6 protein (mCDH6 ECD-his, conventionally synthesized; the mCDH6 ECD sequence was derived from Uniprot# P97326 (19-615)) were diluted with PBS to a final concentration of 1 µg/mL, and added to a 96-well ELISA plate at 100 µL/well. The plate was incubated at 4 °C overnight. The next day, the plate was washed 3 times with a plate washing solution (PBS + 0.01% (v/v) Tween 20), and a blocking buffer (PBS + 0.01% (v/v) Tween 20 + 2% (w/w) BSA) was added for blocking at room temperature for 2 h. The blocking buffer was discarded, and the plate was washed 3 times. The chimeric antibodies were diluted with 1% BSA + PBS (purchased from Cytiva, Cat. No. SH30264.01) and added to the 96-well plate at 50 µL/well. After incubation at 37 °C for 2 h, the plate was washed 3 times with a plate washing solution. Horseradish peroxidase-labeled secondary antibody Peroxidase AffiniPure Goat Anti-Human IgG, Fcy fragment specific (purchased from Jackson Immuno, Cat. No. 109-035-098) was diluted with 1% BSA at a ratio of 1:5000 and added to the 96-well plate at 50 µL/well. The plate was incubated at 37 °C for 1 h and then washed 5 times with a plate washing solution. A TMB substrate (purchased from Seracare, Cat. No. 5120-0077) was added at 50 µL/well and incubated at room temperature for 10 min, then a stop solution (1.0 N HCl) was added at 50 µL/well. OD450 nm values were read using a microplate reader (Biotek). Analysis was performed by the GraphPad Prism software, data were fitted, and EC50 values were calculated. The ELISA results are shown in Table 5. The chimeric antibodies tested all had strong binding to the human CDH6 protein, the monkey CDH6 protein, and the murine CDH6 protein.

**Table 5. Binding activity of chimeric antibodies to human CDH6, monkey CDH6, and murine CDH6 proteins**

| Antibody | Human CDH6-his | | Cyno CDH6-his | | Mouse CDH6-his | |
|---|---|---|---|---|---|---|
| | EC50 (nM) | Max OD450 | EC50 (nM) | Max OD450 | EC50 (nM) | Max OD450 |
| CHI-CDH6-1 | 0.040 | 2.23 | 0.023 | 2.54 | 0.030 | 1.81 |
| CHI-CDH6-2 | 0.027 | 2.07 | 0.022 | 2.12 | 0.023 | 1.62 |
| CHI-CDH6-3 | 0.030 | 2.56 | 0.041 | 2.64 | 0.051 | 2.38 |
| CHI-CDH6-4 | 0.053 | 2.14 | 0.034 | 2.23 | 0.035 | 2.32 |
| CHI-CDH6-5 | 0.043 | 1.79 | 0.046 | 1.96 | 0.046 | 1.92 |
| CHI-CDH6-6 | 0.034 | 2.18 | 0.030 | 2.24 | 0.025 | 1.60 |
| CHI-CDH6-7 | 0.488 | 1.78 | 0.178 | 2.05 | 0.058 | 2.12 |
| CHI-CDH6-8 | 0.033 | 2.31 | 0.026 | 2.12 | 0.028 | 1.82 |
| CHI-CDH6-9 | 0.016 | 2.58 | 0.026 | 2.78 | 0.022 | 2.77 |
| CHI-CDH6-10 | 0.025 | 2.03 | 0.026 | 2.26 | 0.026 | 1.75 |
| CHI-CDH6-11 | 0.034 | 2.24 | 0.032 | 2.32 | 0.040 | 1.77 |
| CHI-CDH6-12 | 0.105 | 1.82 | 0.044 | 2.45 | 0.052 | 2.09 |
| CHI-CDH6-13 | 0.009 | 2.62 | 0.017 | 2.72 | 0.016 | 2.76 |
| CHI-CDH6-14 | 0.044 | 2.50 | 0.039 | 2.55 | 0.049 | 2.48 |
| DS-H01L02 | 0.168 | 1.89 | 0.103 | 2.18 | / | 0.60 |
| NOV0712 | 0.043 | 2.32 | 0.039 | 2.44 | 0.039 | 2.42 |
| hIgG1 | / | 0.07 | / | 0.06 | / | 0.10 |

| | | | | | | |
|---|---|---|---|---|---|---|
| Note: "/" indicates no binding or weak binding, and EC50 values could not be fitted or were > 10 nM. | | | | | | |

### 2.3. Assay on binding of chimeric antibodies to tumor cells expressing CDH6 by flow cytometry assay (FACS)

OVCAR3 cells with high expression of CDH6 and PA-1 cells with moderate expression of CDH6 were used to detect the binding activity of the antibodies to the cells. OVCAR3 cells (purchased from ATCC, # HTB-161) or PA-1 cells (purchased from Nanjing Cobioer Biosciences Co., Ltd., # CBP60800) were cultured until a confluence of 90% was reached, the medium was removed by pipetting, the cells were washed once with PBS, and then treated with an enzyme-free cell dissociation solution (Versene, purchased from Gibco, Cat. No. 15040-066) and the cells were collected. The cells were washed once with PBS, counted, resuspended to 2×10⁶ cells/mL in an FACS buffer (PBS + 2% FBS), and then added to a 96-well plate at 50 µL/well. The chimeric antibodies were diluted with FACS buffer (PBS + 2% FBS) and added to the 96-well plate at 50 µL/well, and the plate was incubated at 4 °C for 1 h. The plate was washed 3 times with PBS, and the secondary antibody Alexa Fluor^{®} 647 AffiniPure Goat Anti-Human IgG, Fcy fragment specific (purchased from Jackson Immuno, Cat. No. 109-605-098) was diluted in a 1:800 ratio with an FACS buffer. The dilution was added to the 96-well plate at 50 µL/well, and the plate was incubated at 4 °C for 1 h. After the plates were centrifuged and washed 2 times with PBS, the cells were resuspended in an FACS buffer. The mean fluorescence intensity (MFI) was measured using FACS Canton II (BD). Analysis was performed by the GraphPad Prism software, data were fitted, and EC50 values were calculated. The FACS results are shown in Table 6. The chimeric antibodies tested had strong binding to both OVCAR3 cells and PA-1 cells.

**Table 6. Binding activity of chimeric antibodies to OVCAR3 and PA-1 cells**

| Antibody | OVCAR3 | | PA-1 | |
|---|---|---|---|---|
| | EC50 (nM) | Max MFI | EC50 (nM) | Max MFI |
| CHI-CDH6-1 | 0.13 | 14731 | 0.10 | 4394 |
| CHI-CDH6-2 | 0.36 | 15631 | 0.20 | 4761 |
| CHI-CDH6-3 | 1.16 | 15511 | 0.13 | 3874 |
| CHI-CDH6-4 | 3.17 | 14010 | 2.48 | 3991 |
| CHI-CDH6-5 | 0.57 | 12698 | 0.49 | 4559 |
| CHI-CDH6-6 | 0.67 | 14787 | 0.43 | 4627 |
| CHI-CDH6-7 | 5.57 | 12036 | 7.57 | 2473 |
| CHI-CDH6-8 | 0.46 | 16086 | 0.23 | 4714 |
| CHI-CDH6-9 | 0.51 | 18231 | 0.16 | 4401 |
| CHI-CDH6-10 | 0.20 | 13915 | 0.08 | 4068 |
| CHI-CDH6-11 | 2.14 | 14540 | 1.78 | 4688 |
| CHI-CDH6-12 | 2.22 | 12495 | 2.27 | 3151 |
| CHI-CDH6-13 | 2.08 | 17632 | 1.97 | 4439 |
| CHI-CDH6-14 | 0.43 | 15671 | 0.26 | 5407 |
| DS-H01L02 | 0.80 | 9732 | 1.09 | 1879 |
| NOV0712 | 1.92 | 11539 | 1.46 | 3595 |
| hIgG1 | / | 63 | / | 64 |

### 2.4. Assay on endocytic activity of chimeric antibodies

OVCAR3 cells were cultured until a confluence of 90% was reached, the medium was removed by pipetting, the cells were washed once with PBS, and then treated with an enzyme-free cell dissociation solution (Versene, purchased from Gibco, Cat. No. 15040-066) and the cells were collected. The cells were washed once with PBS, counted, resuspended to 2×10⁶ cells/mL in an FACS buffer (PBS + 2% FBS), and then added to two 96-well plates at 50 µL/well. The chimeric antibodies were diluted with FACS buffer (PBS + 2% FBS) to 10 µg/mL and separately added to the two 96-well plates at 50 µL/well, and the plates were incubated at 4 °C for 1 h. After the cells were washed 3 times with PBS, the cells were resuspended in an FACS buffer. One 96-well plate was incubated at 4 °C for 1 h, and the other 96-well plate was incubated at 37 °C for 1 h. After the cells were washed 3 times with PBS, a secondary antibody Alexa Fluor^{®} 647 AffiniPure Goat Anti-Human IgG, Fcy fragment specific (purchased from Jackson Immuno, Cat. No. 109-605-098) was added to each well, and the plates were incubated at 4 °C for 1 h. After the plates were centrifuged and washed 2 times with PBS, the cells were resuspended in an FACS buffer. The mean fluorescence intensity (MFI) was measured using FACS Canton II (BD). The results are shown in Table 7. The chimeric antibodies tested all had good endocytic activity.

**Table 7. Endocytic activity of chimeric antibodies**

| Antibody | MFI_4 °C | MFI_37 °C | Absolute endocytosis value (MFI_4 °C-MFI_37 °C) | Endocytosis percentage (MFI_4 °C-MFI_37 °C)/ MFI_4 °Cx100% |
|---|---|---|---|---|
| CHI-CDH6-1 | 10635 | 1946 | 8689 | 82% |
| CHI-CDH6-2 | 11115 | 3122 | 7993 | 72% |
| CHI-CDH6-3 | 9276 | 2161 | 7115 | 77% |
| CHI-CDH6-4 | 9515 | 2216 | 7299 | 77% |
| CHI-CDH6-5 | 8806 | 2861 | 5944 | 68% |
| CHI-CDH6-6 | 9934 | 1250 | 8684 | 87% |
| CHI-CDH6-7 | 5584 | 483 | 5101 | 91% |
| CHI-CDH6-8 | 10102 | 1671 | 8431 | 83% |
| CHI-CDH6-9 | 10996 | 2864 | 8132 | 74% |
| CHI-CDH6-10 | 10067 | 1414 | 8654 | 86% |
| CHI-CDH6-11 | 10377 | 2990 | 7387 | 71% |
| CHI-CDH6-12 | 5743 | 662 | 5081 | 88% |
| CHI-CDH6-13 | 5972 | 2086 | 3885 | 65% |
| CHI-CDH6-14 | 9234 | 2358 | 6876 | 74% |
| DS-H01L02 | 3833 | 589 | 3244 | 85% |
| NOV0712 | 6515 | 2196 | 4319 | 66% |

### 2.5. Assay on affinity of chimeric antibodies for human CDH6 protein by Biacore

The affinity of the antibody for the antigen was determined by a multi-cycle kinetic method using a BIAcore 8K instrument. In each cycle, the antibody to be tested was captured using a Protein A chip (purchased from GE, Cat. No. 29-1275-56), and then the antigen human CDH6 protein (hCDH6-his, conventionally synthesized) was injected. The association and dissociation processes of the antibody with the antigen protein were recorded, and finally, the chip was regenerated using Glycine pH 1.5, wherein the mobile phase was HBS-EP+ (10 mM HEPES, 150 mM NaCl, 3 mM EDTA, 0.05% surfactant P20, and 1 mM Ca), the flow rate was 30 µL/min, the association time was 240 s, the dissociation time was 300 s, the regeneration time was 30 s, the detection temperature was 25 °C, and the antigen concentration was 31.25-1000 nM. The data were analyzed using Biacore 8K analysis software (version No. 2.0) based on the 1:1 binding model to fit the antibody-antigen binding kinetic parameters, including the association rate constant ka, the dissociation rate constant kd, and the equilibrium dissociation constant KD. The results are shown in Table 8. The affinity values of the control antibody DS-H01L02 were undetectable. Antibodies not shown in the table had undetectable affinity values.

**Table 8. Affinity of chimeric antibodies for human CDH6 protein**

| Antibody | ka (1/Ms) | kd (1/s) | KD (M) |
|---|---|---|---|
| CHI-CDH6-1 | 6.59E+05 | 5.85E-03 | 8.87E-09 |
| CHI-CDH6-2 | 5.03E+05 | 1.85E-03 | 3.68E-09 |
| CHI-CDH6-6 | 8.68E+04 | 5.08E-03 | 5.86E-08 |
| CHI-CDH6-8 | 1.21E+05 | 3.78E-03 | 3.12E-08 |
| CHI-CDH6-9 | 1.16E+04 | 1.59E-03 | 1.38E-07 |
| CHI-CDH6-10 | 1.79E+05 | 3.64E-03 | 2.03E-08 |
| CHI-CDH6-11 | 3.52E+04 | 2.01E-04 | 5.72E-09 |
| CHI-CDH6-13 | 2.36E+04 | 1.84E-03 | 7.81E-08 |
| NOV0712 | 4.99E+04 | 2.67E-03 | 5.35E-08 |
| DS-H01L02 | / | / | / |

| | | | |
|---|---|---|---|
| Note: "/" indicates that no value could be detected. | | | |

### Example 3. Humanization of Antibody

By alignment with the IMGT database (http://imgt.cines.fr) for germline genes from heavy and light chain variable regions of human antibodies, germline genes, with high homology with the murine antibodies, from heavy and light chain variable regions were selected as templates, and CDRs of the murine antibodies divided according to Kabat were separately grafted into corresponding human templates to give variable region sequences in the order of FR1-CDR1-FR2-CDR2-FR3-CDR3-FR4. Based on the three-dimensional structure of the antibody, embedded residues, residues directly interacting with the CDRs, and residues in framework regions that had an important influence on the conformation of VL and VH were back-mutated, thus giving humanized monoclonal antibodies.

### 3.1 Humanization of CDH6-1

For murine antibody CDH6-1, the humanized light chain templates were IGKV4-1*01/IGKV1-33*01 and IGKJ4*01, and the humanized heavy chain templates were IGHV1-69*02 and IGHJ6*01. The CDRs of murine antibody CDH6-1 were separately grafted into their human templates with the CDR sequences unchanged, so as to give the corresponding humanized antibody hCDH6-1. Key amino acids in FR sequences of the humanized antibodies of hCDH6-1 were back-mutated to amino acids corresponding to the murine antibody as needed to ensure the original affinity. Detailed back mutation design is shown in Table 9.

**Table 9. Back mutation design for humanized antibodies of hCDH6-1**

| **VL** | | **VH** | |
|---|---|---|---|
| L1 | Graft(IGKV4-1*01) + G72E | H1 | Graft(IGHV1-69*02) + G27Y,A72V |
| L2 | Graft(IGKV4-1*01) + P44S,G72E | H2 | Graft(IGHV1-69*02) + G27Y,S30T,A72V |
| L3 | Graft(IGKV 1-33 *01) + A47P,G72E | H3 | Graft(IGHV1-69*02) + G27Y,S30T,I70L,A72V |
| | | H4 | Graft(IGHV1-69*02) + G27Y,S30T,A40R,A72V |

| | | | |
|---|---|---|---|
| Note: Graft denotes that the CDRs of the murine antibody are grafted into the human germline template FR sequences; G72E denotes that G at position 72 of Graft is mutated to E, and so on for others. The back-mutated amino acids are numbered in the natural order. | | | |

Specific sequences of the variable regions of the hCDH6-1 humanized antibodies are as follows (the mutations are shown in bold):
hCDH6-1.VL1 has an amino acid sequence set forth in SEQ ID NO: 133: 
DIVMTQSPDSLAVSLGERATINCRASESVSIHGTHLMHWYQQKPGQPPKLLIYAASNLESGV
PDRFSGSGSETDFTLTISSLQAEDVAVYYCQQSFEDPWTFGGGTKVEIK
hCDH6-1.VL2 has an amino acid sequence set forth in SEQ ID NO: 134: 
DIVMTQSPDSLAVSLGERATINCRASESVSIHGTHLMHWYQQKSGQPPKLLIYAASNLESGV
PDRFSGSGSETDFTLTISSLQAEDVAVYYCQQSFEDPWTFGGGTKVEIK
hCDH6-1.VL3 has an amino acid sequence set forth in SEQ ID NO: 135: 
DIQMTQSPSSLSASVGDRVTITCRASESVSIHGTHLMHWYQQKPGKPPKLLIYAASNLESGV
PSRFSGSGSETDFTFTISSLQPEDIATYYCQQSFEDPWTFGGGTKVEIK
hCDH6-1.VH1 has an amino acid sequence set forth in SEQ ID NO: 136: 
EVQLVQSGAEVKKPGSSVKVSCKASGYTFSNYWMHWVRQAPGQGLEWMGDIDPSHSETH
YTQKFKDRVTITVDKSTSTAYMELSSLRSEDTAVYYCARGDLGRFDYWGQGTTVTVSS
hCDH6-1.VH2 has an amino acid sequence set forth in SEQ ID NO: 137: 
EVQLVQSGAEVKKPGSSVKVSCKASGYTFTNYWMHWVRQAPGQGLEWMGDIDPSHSET
HYTQKFKDRVTITVDKSTSTAYMELSSLRSEDTAVYYCARGDLGRFDYWGQGTTVTVSS
hCDH6-13.VH3 has an amino acid sequence set forth in SEQ ID NO: 138: 
EVQLVQSGAEVKKPGSSVKVSCKASGYTFTNYWMHWVRQAPGQGLEWMGDIDPSHSET
HYTQKFKDRVTLTVDKSTSTAYMELSSLRSEDTAVYYCARGDLGRFDYWGQGTTVTVSS
hCDH6-1.VH4 has an amino acid sequence set forth in SEQ ID NO: 139:

EVQLVQSGAEVKKPGSSVKVSCKASGYTFTNYWMHWVRQRPGQGLEWMGDIDPSHSET
HYTQKFKDRVTITVDKSTSTAYMELSSLRSEDTAVYYCARGDLGRFDYWGQGTTVTVSS

The human germline light chain template IGKV4-1*01 has an amino acid sequence set forth in SEQ ID NO: 140:

DIVMTQSPDSLAVSLGERATINCKSSQSVLYSSNNKNYLAWYQQKPGQPPKLLIYWASTRES
GVPDRFSGSGSGTDFTLTISSLQAEDVAVYYCQQYYSTP

The human germline light chain template IGKV1-33*01 has an amino acid sequence set forth in SEQ ID NO: 141:

DIQMTQSPSSLSASVGDRVTITCQASQDISNYLNWYQQKPGKAPKLLIYDASNLETGVPSRF
SGSGSGTDFTFTISSLQPEDIATYYCQQYDNLP

The human germline light chain template IGKJ4*01 has an amino acid sequence set forth in SEQ ID NO: 142:
FGGGTKVEIK

The human germline heavy chain template IGHV1-69*02 has an amino acid sequence set forth in SEQ ID NO: 143:

QVQLVQSGAEVKKPGSSVKVSCKASGGTFSSYTISWVRQAPGQGLEWMGRIIPILGIANYA
QKFQGRVTITADKSTSTAYMELSSLRSEDTAVYYCAR

The human germline heavy chain template IGHJ6*01 has an amino acid sequence set forth in SEQ ID NO: 144:
WGQGTTVTVSS

From the back mutation design for the light chain and heavy chain variable regions of humanized antibodies of hCDH6-1, different light chain and heavy chain sequences were selected for cross combination to finally give a plurality of humanized antibodies of hCDH6-1. The amino acid sequences of the variable regions of the antibodies are as follows:

**Table 10. Corresponding amino acid sequences of variable regions of antibodies of hCDH6-1**

| Fv | hCDH6-1.VH1 | hCDH6-1.VH2 | hCDH6-1.VH3 | hCDH6-1.VH4 |
|---|---|---|---|---|
| hCDH6-1.VL1 | hCDH6-1-H1L1 | hCDH6-1-H2L1 | hCDH6-1-H3L1 | hCDH6-1-H4L1 |
| hCDH6-1.VL2 | hCDH6-1-H1L2 | hCDH6-1-H2L2 | hCDH6-1-H3L2 | hCDH6-1-H4L2 |
| hCDH6-1.VL3 | hCDH6-1-H1L3 | hCDH6-1-H2L3 | hCDH6-1-H3L3 | hCDH6-1-H4L3 |

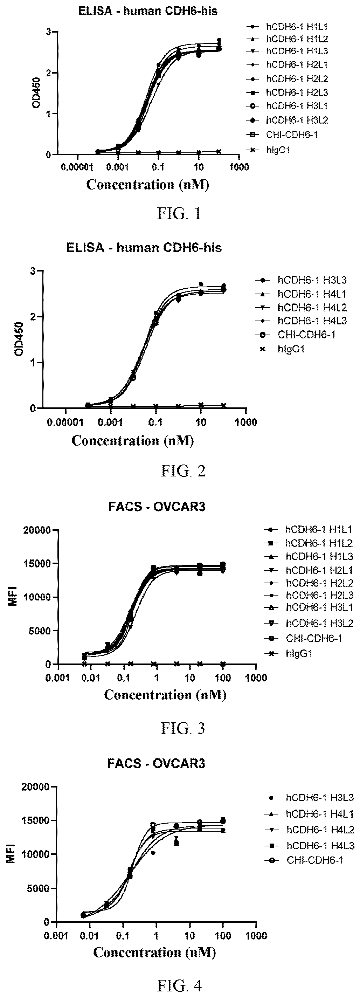

According to the above sequences, heavy chain (HC) and light chain (LC) plasmids were constructed (the nucleic acid sequences encoding the VH and VL of the antibodies were recombined into an expression vector carrying a signal peptide (SEQ ID NO: 132, MGWSWILLFLLSVTAGVHS) and a heavy chain constant region/light chain constant region sequence to obtain recombinant plasmids expressing VH-CH1-Fc/VL-CL). According to the expression and purification methods in Example 2, the hCDH6-1 humanized antibodies were obtained, and the binding activity of the humanized antibodies was detected by ELISA and FACS (see 2.2 and 2.3 for the method). The results are shown in Table 11 and FIGs. 1-4. The binding activity of the hCDH6-1 humanized antibodies to human CDH6 protein and OVCAR3 cells was comparable to that of the chimeric antibodies.

**Table 11. Binding of hCDH6-1 humanized antibodies to human CDH6 protein and OVCAR3 cells**

| **Antibody** | **hCDH6-his** | | **OVCAR3** | |
|---|---|---|---|---|
| | **EC50 (nM)** | **Max OD450** | **EC50 (nM)** | **Max MFI** |
| hCDH6-1-H1L1 | 0.025 | 2.80 | 0.166 | 14359 |
| hCDH6-1-H1L2 | 0.027 | 2.75 | 0.177 | 14530 |
| hCDH6-1-H1L3 | 0.029 | 2.67 | 0.175 | 14515 |
| hCDH6-1-H2L1 | 0.046 | 2.65 | 0.228 | 14478 |
| hCDH6-1-H2L2 | 0.026 | 2.63 | 0.147 | 14279 |
| hCDH6-1-H2L3 | 0.029 | 2.61 | 0.17 | 14761 |
| hCDH6-1-H3L1 | 0.026 | 2.60 | 0.154 | 14632 |
| hCDH6-1-H3L2 | 0.026 | 2.58 | 0.167 | 14298 |
| hCDH6-1-H3L3 | 0.030 | 2.71 | 0.155 | 14583 |
| hCDH6-1-H4L1 | 0.031 | 2.62 | 0.152 | 13974 |
| hCDH6-1-H4L2 | 0.026 | 2.55 | 0.176 | 15205 |
| hCDH6-1-H4L3 | 0.029 | 2.61 | 0.162 | 15139 |
| CHI- hCDH6-1 | 0.032 | 2.59 | 0.194 | 14886 |

Four hCDH6-1 humanized antibodies with fewer back mutations were selected, and the affinity for human CDH6 protein was further confirmed using Biacore. The results are shown in Table 12, indicating that the affinity of the selected hCDH6-1 humanized antibodies was comparable to that of the chimeric antibodies.

**Table 12. Affinity of hCDH6-1 humanized antibody for human CDH6 protein**

| **Antibody** | **ka (1/Ms)** | **kd (1/s)** | **KD (M)** |
|---|---|---|---|
| hCDH6-1-H1L1 | 2.10E+03 | 4.56E-03 | 2.17E-08 |
| hCDH6-1-H1L2 | 1.96E+05 | 4.22E-03 | 2.15E-08 |
| hCDH6-1-H1L3 | 4.35E+05 | 8.24E-03 | 1.89E-08 |
| hCDH6-1-H2L3 | 4.57E+05 | 8.21E-03 | 1.80E-08 |
| CHI- hCDH6-1 | 4.42E+05 | 5.04E-03 | 1.14E-08 |

### 3.2 Humanization of CDH6-6

For murine antibody CDH6-6, the humanized light chain templates were IGKV1-NL1*01/IGKV2-28*01 and IGKJ2*01, and the humanized heavy chain templates were IGHV1-46*01 and IGHJ6*01. The CDRs of murine antibody CDH6-6 were separately grafted into their human templates with the CDR sequences unchanged, so as to give the corresponding humanized version hCDH6-6. Key amino acids in FR sequences of the humanized antibodies of hCDH6-6 were back-mutated to amino acids corresponding to the murine antibody as needed to ensure the original affinity. Detailed back mutation design is shown in Table 13.

**Table 13. Back mutation design for humanized antibodies of hCDH6-6**

| **VL** | | **VH** | |
|---|---|---|---|
| L1 | Graft(IGKV1-NL1*01) + A43S | H1 | Graft(IGHV1-46*01) + R72V,T74K |
| L2 | Graft(IGKV1-NL1*01) + A43S,L48V | H2 | Graft(IGHV1-46*01) + M70L,R72V,T74K |
| L3 | Graft(IGKV1-NL1*01) + A43S,K45Q,L48V | H3 | Graft(IGHV1-46*01) + G42R,R72V,T74K |
| L4 | Graft(IGKV1-NL1*01) + A43S,L48V,T85R | | |
| L5 | Graft(IGKV2-28*01) + Q42K,I48V | | |

| | | | |
|---|---|---|---|
| Note: Graft denotes that the CDRs of the murine antibody are grafted into the human germline template FR sequences; A43S denotes that A at position 43 of Graft is mutated to S, and so on for others. The back-mutated amino acids are numbered in the natural order. | | | |

Specific sequences of the variable regions of the hCDH6-6 humanized antibodies are as follows (the mutations are shown in bold):
hCDH6-6.VL1 has an amino acid sequence set forth in SEQ ID NO: 145: 
DIQMTQSPSSLSASVGDRVTITCRASENIDSYLAWYQQKPGKSPKLLLYSATLLADGVPSRFS
GSGSGTDYTLTISSLQPEDFATYYCQHYYRTPPTFGQGTKLEIK
hCDH6-6.VL2 has an amino acid sequence set forth in SEQ ID NO: 146: 
DIQMTQSPSSLSASVGDRVTITCRASENIDSYLAWYQQKPGKSPKLLVYSATLLADGVPSRF
SGSGSGTDYTLTISSLQPEDFATYYCQHYYRTPPTFGQGTKLEIK
hCDH6-6.VL3 has an amino acid sequence set forth in SEQ ID NO: 147: 
DIQMTQSPSSLSASVGDRVTITCRASENIDSYLAWYQQKPGKSPQLLVYSATLLADGVPSRF
SGSGSGTDYTLTISSLQPEDFATYYCQHYYRTPPTFGQGTKLEIK
hCDH6-6.VL4 has an amino acid sequence set forth in SEQ ID NO: 148: 
DIQMTQSPSSLSASVGDRVTITCRASENIDSYLAWYQQKPGKSPKLLVYSATLLADGVPSRF
SGSGSGTDYTLTISSLQPEDFARYYCQHYYRTPPTFGQGTKLEIK
hCDH6-6.VL5 has an amino acid sequence set forth in SEQ ID NO: 149: 
DIVMTQSPLSLPVTPGEPASISCRASENIDSYLAWYLQKPGKSPQLLVYSATLLADGVPDRFS
GSGSGTDFTLKISRVEAEDVGVYYCQHYYRTPPTFGQGTKLEIK
hCDH6-6.VH1 has an amino acid sequence set forth in SEQ ID NO: 150: 
EVQLVQSGAEVKKPGASVKVSCKASGYTFTDYYMAWVRQAPGQGLEWMGYIFPNSGGT
DYSQKFKGRVTMTVDKSTSTVYMELSSLRSEDTAVYYCARELGADYGDYYAMDHWGQG
TTVTVSS
hCDH6-6.VH2 has an amino acid sequence set forth in SEQ ID NO: 151: 
EVQLVQSGAEVKKPGASVKVSCKASGYTFTDYYMAWVRQAPGQGLEWMGYIFPNSGGT
DYSQKFKGRVTLTVDKSTSTVYMELSSLRSEDTAVYYCARELGADYGDYYAMDHWGQGT
TVTVSS
hCDH6-6.VH3 has an amino acid sequence set forth in SEQ ID NO: 152:

EVQLVQSGAEVKKPGASVKVSCKASGYTFTDYYMAWVRQAPRQGLEWMGYIFPNSGGT
DYSQKFKGRVTMTVDKSTSTVYMELSSLRSEDTAVYYCARELGADYGDYYAMDHWGQG
TTVTVSS

The human germline light chain template IGKV1-NL1*01 has an amino acid sequence set forth in SEQ ID NO: 153:

DIQMTQSPSSLSASVGDRVTITCRASQGISNSLAWYQQKPGKAPKLLLYAASRLESGVPSRFS

GSGSGTDYTLTISSLQPEDFATYYCQQYYSTP

The human germline light chain template IGKV2-28*01 has an amino acid sequence set forth in SEQ ID NO: 154:

DIVMTQSPLSLPVTPGEPASISCRSSQSLLHSNGYNYLDWYLQKPGQSPQLLIYLGSNRASG
VPDRFSGSGSGTDFTLKISRVEAEDVGVYYCMQALQTP

The human germline light chain template IGKJ2*01 has an amino acid sequence set forth in SEQ ID NO: 155:
FGQGTKLEIK

The human germline heavy chain template IGHV1-46*01 has an amino acid sequence set forth in SEQ ID NO: 156:

QVQLVQSGAEVKKPGASVKVSCKASGYTFTSYYMHWVRQAPGQGLEWMGIINPSGGSTS
YAQKFQGRVTMTRDTSTSTVYMELSSLRSEDTAVYYCAR

The human germline heavy chain template IGHJ6*01 has an amino acid sequence set forth in SEQ ID NO: 144:
WGQGTTVTVSS

From the back mutation design for the light chain and heavy chain variable regions of humanized antibodies of hCDH6-6, different light chain and heavy chain sequences were selected for cross combination to finally give a plurality of humanized antibodies of hCDH6-6. The amino acid sequences of the variable regions of the antibodies are as follows:

**Table 14. Corresponding amino acid sequences of variable regions of antibodies of hCDH6-6**

| Fv | hCDH6-6.VH1 | hCDH6-6.VH2 | hCDH6-6.VH3 |
|---|---|---|---|
| hCDH6-6.VL1 | hCDH6-6-H1L1 | hCDH6-6-H2L1 | hCDH6-6-H3L1 |
| hCDH6-6.VL2 | hCDH6-6-H1L2 | hCDH6-6-H2L2 | hCDH6-6-H3L2 |
| hCDH6-6.VL3 | hCDH6-6-H1L3 | hCDH6-6-H2L3 | hCDH6-6-H3L3 |
| hCDH6-6.VL4 | hCDH6-6-H1L4 | hCDH6-6-H2L4 | hCDH6-6-H3L4 |
| hCDH6-6.VL5 | hCDH6-6-H1L5 | hCDH6-6-H2L5 | hCDH6-6-H3L5 |

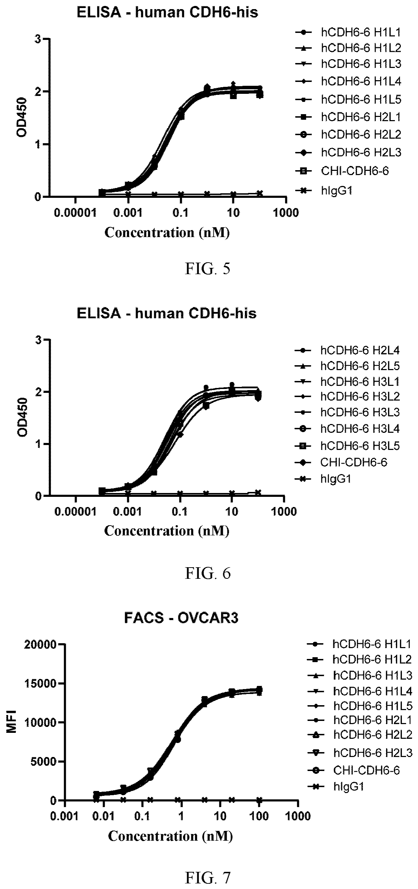

According to the above sequences, heavy chain (HC) and light chain (LC) plasmids were constructed (the nucleic acid sequences encoding the VH and VL of the antibodies were recombined into an expression vector carrying a signal peptide (SEQ ID NO: 132, MGWSWILLFLLSVTAGVHS) and a heavy chain constant region/light chain constant region sequence to obtain recombinant plasmids expressing VH-CH1-Fc/VL-CL). According to the expression and purification methods in Example 2, the hCDH6-6 humanized antibodies were obtained, and the binding activity of the humanized antibodies was detected by ELISA and FACS (see 2.2 and 2.3 for the method). The results are shown in Table 15 and FIGs. 5-8. The binding activity of the hCDH6-6 humanized antibodies to human CDH6 protein and OVCAR3 cells was comparable to that of the chimeric antibodies.

**Table 15. Binding of hCDH6-6 humanized antibodies to human CDH6 protein and OVCAR3 cells**

| Antibody | **hCDH6-his** | | **OVCAR3** | |
|---|---|---|---|---|
| | **EC50 (nM)** | **Max OD450** | **EC50 (nM)** | **Max MFI** |
| hCDH6-6-H1L 1 | 0.037 | 2.08 | 0.62 | 13708 |
| hCDH6-6-H1L2 | 0.028 | 2.07 | 0.62 | 14269 |
| hCDH6-6-H1L3 | 0.031 | 1.99 | 0.63 | 13774 |
| hCDH6-6-H1L4 | 0.021 | 2.16 | 0.64 | 14370 |
| hCDH6-6-H1L5 | 0.031 | 2.06 | 0.61 | 14176 |
| hCDH6-6-H2L 1 | 0.032 | 2.01 | 0.61 | 14040 |
| hCDH6-6-H2L2 | 0.026 | 1.97 | 0.56 | 14283 |
| hCDH6-6-H2L3 | 0.025 | 2.09 | 0.57 | 14106 |
| hCDH6-6-H2L4 | 0.029 | 2.14 | 0.62 | 14345 |
| hCDH6-6-H2L5 | 0.026 | 2.10 | 0.64 | 14588 |
| hCDH6-6-H3L 1 | 0.050 | 2.02 | 0.87 | 13736 |
| hCDH6-6-H3L2 | 0.030 | 2.09 | 0.85 | 14592 |
| hCDH6-6-H3L3 | 0.031 | 2.00 | 0.74 | 14280 |
| hCDH6-6-H3L4 | 0.041 | 2.05 | 0.70 | 14502 |
| hCDH6-6-H3L5 | 0.043 | 1.97 | 0.69 | 14234 |
| CHI- hCDH6-6 | 0.028 | 2.02 | 0.68 | 14124 |

Four hCDH6-6 humanized antibodies with fewer back mutations were selected, and the affinity for human CDH6 protein was further confirmed using Biacore. The results are shown in Table 16, indicating that the affinity of the four selected hCDH6-6 humanized antibodies was comparable to that of the chimeric antibodies.

**Table 16. Affinity of hCDH6-6 humanized antibody for human CDH6 protein**

| **Antibody** | **ka (1/Ms)** | **kd (1/s)** | **KD (M)** |
|---|---|---|---|
| hCDH6-6-H1L1 | 3.86E+04 | 7.43E-03 | 1.92E-07 |
| hCDH6-6-H1L2 | 4.41E+04 | 6.07E-03 | 1.38E-07 |
| hCDH6-6-H2L1 | 4.62E+04 | 6.16E-03 | 1.33E-07 |
| hCDH6-6-H2L2 | 5.21E+04 | 4.68E-03 | 8.98E-08 |
| CHI- hCDH6-6 | 4.87E+04 | 4.24E-03 | 8.71E-08 |

### 3.3 Humanization of CDH6-14

For murine antibody CDH6-14, the humanized light chain templates were IGKV1-33*01/IGKV4-1*01 and IGKJ4*01, and the humanized heavy chain templates were IGHV1-3*01 and IGHJ6*01. The CDRs of murine antibody CDH6-14 were separately grafted into their human templates with the CDR sequences unchanged, so as to give the corresponding humanized version hCDH6-14. Key amino acids in FR sequences of the humanized antibodies of hCDH6-14 were back-mutated to amino acids corresponding to the murine antibody as needed to ensure the original affinity. Detailed back mutation design is shown in Table 17.

**Table 17. Back mutation design for humanized antibodies of hCDH6-14**

| **VL** | | **VH** | |
|---|---|---|---|
| L1 | Graft(IGKV1-33*01) + | H1 | Graft(IGHV1-3*01) + R72V,R98S |
| | P44F,Y49S | | |
| L2 | Graft(IGKV1-33*01) + K42G,Y49S | H2 | Graft(IGHV1-3*01) + V2I,R72V,R98S |
| L3 | Graft(IGKV4-1*01) + P43T,Y49S | H3 | Graft(IGHV1-3*01) + V2I,I70L,R72V,R98S |
| | | H4 | Graft(IGHV1-3*01) + R44G,R72V,R98S |
| | | H5 | Graft(IGHV1-3*01) + V5Q,R72V,R98S |
| | | H6 | Graft(IGHV1-3*01) + R72V,Y95L,R98S |

| | | | |
|---|---|---|---|
| Note: Graft denotes that the CDRs of the murine antibody are grafted into the human germline template FR sequences; P44F denotes that P at position 44 of Graft is mutated to F, and so on for others. The back-mutated amino acids are numbered in the natural order. | | | |

Specific sequences of the variable regions of the hCDH6-14 humanized antibodies are as follows (the mutations are shown in bold):
hCDH6-14.VL1 has an amino acid sequence set forth in SEQ ID NO: 157: 
DIQMTQSPSSLSASVGDRVTITCRASQDIRNYLNWYQQKPGKAFKLLISYTSRLHSGVPSRF
SGSGSGTDFTFTISSLQPEDIATYYCQQYSQLPWTFGGGTKVEIK
hCDH6-14.VL2 has an amino acid sequence set forth in SEQ ID NO: 158: 
DIQMTQSPSSLSASVGDRVTITCRASQDIRNYLNWYQQKPGGAPKLLISYTSRLHSGVPSRF
SGSGSGTDFTFTISSLQPEDIATYYCQQYSQLPWTFGGGTKVEIK
hCDH6-14.VL3 has an amino acid sequence set forth in SEQ ID NO: 159: 
DIVMTQSPDSLAVSLGERATINCRASQDIRNYLNWYQQKPGQTPKLLISYTSRLHSGVPDRF
SGSGSGTDFTLTISSLQAEDVAVYYCQQYSQLPWTFGGGTKVEIK
hCDH6-14.VH1 has an amino acid sequence set forth in SEQ ID NO: 160: 
EVQLVQSGAEVKKPGASVKVSCKASGYTFTDNYINWVRQAPGQRLEWMGWIYPGTGNSY
YNEKFKGRVTITVDTSASTAYMELSSLRSEDTAVYYCASSYGDSFDYWGQGTTVTVSS
hCDH6-14.VH2 has an amino acid sequence set forth in SEQ ID NO: 161: 
EIQLVQSGAEVKKPGASVKVSCKASGYTFTDNYINWVRQAPGQRLEWMGWIYPGTGNSY
YNEKFKGRVTITVDTSASTAYMELSSLRSEDTAVYYCASSYGDSFDYWGQGTTVTVSS
hCDH6-14.VH3 has an amino acid sequence set forth in SEQ ID NO: 162: 
EIQLVQSGAEVKKPGASVKVSCKASGYTFTDNYINWVRQAPGQRLEWMGWIYPGTGNSY
YNEKFKGRVTLTVDTSASTAYMELSSLRSEDTAVYYCASSYGDSFDYWGQGTTVTVSS
hCDH6-14.VH4 has an amino acid sequence set forth in SEQ ID NO: 163: 
EVQLVQSGAEVKKPGASVKVSCKASGYTFTDNYINWVRQAPGQGLEWMGWIYPGTGNSY
YNEKFKGRVTITVDTSASTAYMELSSLRSEDTAVYYCASSYGDSFDYWGQGTTVTVSS
hCDH6-14.VH5 has an amino acid sequence set forth in SEQ ID NO: 164: 
EVQLQQSGAEVKKPGASVKVSCKASGYTFTDNYINWVRQAPGQRLEWMGWIYPGTGNS
YYNEKFKGRVTITVDTSASTAYMELSSLRSEDTAVYYCASSYGDSFDYWGQGTTVTVSS
hCDH6-14.VH6 has an amino acid sequence set forth in SEQ ID NO: 165:

EVQLVQSGAEVKKPGASVKVSCKASGYTFTDNYINWVRQAPGQRLEWMGWIYPGTGNSY

YNEKFKGRVTITVDTSASTAYMELSSLRSEDTAVYLCASSYGDSFDYWGQGTTVTVSS

The human germline light chain template IGKV1-33*01 has an amino acid sequence set forth in SEQ ID NO: 141:

DIQMTQSPSSLSASVGDRVTITCQASQDISNYLNWYQQKPGKAPKLLIYDASNLETGVPSRF
SGSGSGTDFTFTISSLQPEDIATYYCQQYDNLP

The human germline light chain template IGKV4-1*01 has an amino acid sequence set forth in SEQ ID NO: 140:

DIVMTQSPDSLAVSLGERATINCKSSQSVLYSSNNKNYLAWYQQKPGQPPKLLIYWASTRES
GVPDRFSGSGSGTDFTLTISSLQAEDVAVYYCQQYYSTP

The human germline light chain template IGKJ4*01 has an amino acid sequence set forth in SEQ ID NO: 142:
FGGGTKVEIK

The human germline heavy chain template IGHV1-3*01 has an amino acid sequence set forth in SEQ ID NO: 166:

QVQLVQSGAEVKKPGASVKVSCKASGYTFTSYAMHWVRQAPGQRLEWMGWINAGNGNT
KYSQKFQGRVTITRDTSASTAYMELSSLRSEDTAVYYCAR

The human germline heavy chain template IGHJ6*01 has an amino acid sequence set forth in SEQ ID NO: 144:
WGQGTTVTVSS

From the back mutation design for the light chain and heavy chain variable regions of humanized antibodies of hCDH6-14, different light chain and heavy chain sequences were selected for cross combination to finally give a plurality of humanized antibodies of hCDH6-14. The amino acid sequences of the variable regions of the antibodies are as follows:

**Table 18. Corresponding amino acid sequences of variable regions of antibodies of hCDH6-14**

| Fv | hCDH6-14.VH 1 | hCDH6-14.VH 2 | hCDH6-14.VH 3 | hCDH6-14.VH 4 | hCDH6-14.VH 5 | hCDH6-14.VH 6 |
|---|---|---|---|---|---|---|
| hCDH6-14.VL 1 | hCDH6-14-H1 L1 | hCDH6-14-H2 L1 | hCDH6-14-H3 L1 | hCDH6-14-H4 L1 | hCDH6-14-H5 L1 | hCDH6-14-H6 L1 |
| hCDH6-14.VL 2 | hCDH6-14-H1 L2 | hCDH6-14-H2 L2 | hCDH6-14-H3 L2 | hCDH6-14-H4 L2 | hCDH6-14-H5 L2 | hCDH6-14-H6 L2 |
| hCDH6-14.VL 3 | hCDH6-14-H1 L3 | hCDH6-144-H 2L3 | hCDH6-14-H3 L3 | hCDH6-14-H4 L3 | hCDH6-14-H5 L3 | hCDH6-14-H6 L3 |

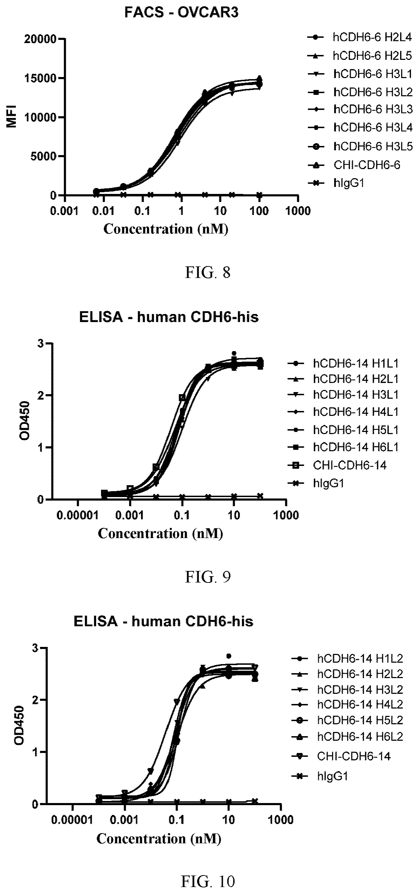

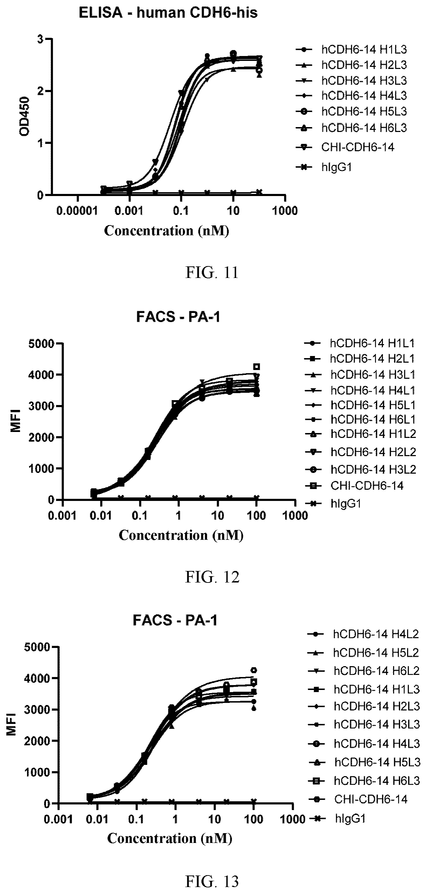

According to the above sequences, heavy chain (HC) and light chain (LC) plasmids were constructed (the nucleic acid sequences encoding the VH and VL of the antibodies were recombined into an expression vector carrying a signal peptide (SEQ ID NO: 132, MGWSWILLFLLSVTAGVHS) and a heavy chain constant region/light chain constant region sequence to obtain recombinant plasmids expressing VH-CH1-Fc/VL-CL). According to the expression and purification methods in Example 2, the hCDH6-14 humanized antibodies were obtained, and the binding activity of the humanized antibodies was detected by ELISA and FACS (see 2.2 and 2.3 for the method). The results are shown in Table 19 and FIGs. 9-13. The binding activity of the hCDH6-14 humanized antibodies to human CDH6 protein and PA-1 cells was comparable to that of the chimeric antibodies.

**Table 19. Binding of hCDH6-14 humanized antibodies to human CDH6 protein and PA-1 cells**

| Antibody | hCDH6-his | | PA-1 | |
|---|---|---|---|---|
| | EC50 (nM) | Max OD450 | EC50 (nM) | Max MFI |
| hCDH6-14-H1L1 | 0.059 | 2.692 | 0.26 | 3679 |
| hCDH6-14-H2L1 | 0.076 | 2.647 | 0.25 | 3561 |
| hCDH6-14-H3L1 | 0.096 | 2.617 | 0.26 | 3488 |
| hCDH6-14-H4L1 | 0.052 | 2.616 | 0.25 | 3825 |
| hCDH6-14-H5L1 | 0.079 | 2.811 | 0.25 | 3784 |
| hCDH6-14-H6L1 | 0.067 | 2.701 | 0.24 | 3705 |
| hCDH6-14-H1L2 | 0.128 | 2.843 | 0.22 | 3604 |
| hCDH6-14-H2L2 | 0.098 | 2.541 | 0.27 | 3927 |
| hCDH6-14-H3L2 | 0.074 | 2.634 | 0.23 | 3486 |
| hCDH6-14-H4L2 | 0.089 | 2.643 | 0.26 | 3569 |
| hCDH6-14-H5L2 | 0.109 | 2.583 | 0.23 | 3321 |
| hCDH6-14-H6L2 | 0.093 | 2.615 | 0.26 | 3536 |
| hCDH6-14-H1L3 | 0.056 | 2.68 | 0.27 | 3578 |
| hCDH6-14-H2L3 | 0.090 | 2.478 | 0.22 | 3783 |
| hCDH6-14-H3L3 | 0.113 | 2.564 | 0.19 | 3467 |
| hCDH6-14-H4L3 | 0.084 | 2.668 | 0.27 | 3872 |
| hCDH6-14-H5L3 | 0.092 | 2.723 | 0.21 | 3601 |
| hCDH6-14-H6L3 | 0.062 | 2.724 | 0.25 | 3890 |
| CHI- hCDH6-14 | 0.034 | 2.649 | 0.27 | 4257 |

### 3.4. Assay on endocytic activity of humanized antibodies

Some of the antibodies were selected for the endocytic activity assay, and the method for assaying the endocytic activity of the humanized antibodies in OVCAR3 cells was the same as that in 2.4. The results are shown in Table 20. The humanized antibodies tested all had good endocytic activity.

**Table 20. Endocytic activity of humanized antibodies**

| Antibody | MFI_4 °C | MFI_37 °C | Absolute endocytosis value (MFI_4 °C-MFI_37 °C) | Endocytosis percentage (MFI_4 °C-MFI_37 °C)/ MFI_4 °C×100% |
|---|---|---|---|---|
| hCDH6-1-H2L3 | 20162 | 3426 | 16736 | 83% |
| hCDH6-6-H2L2 | 20530 | 3098 | 17432 | 85% |
| hCDH6-14-H6L3 | 16119 | 3309 | 12810 | 79% |
| DS-H01L02 | 11434 | 2318 | 9116 | 80% |
| NOV0712 | 13567 | 5812 | 7755 | 57% |

### Example 4. Epitope Identification of CDH6 Antibodies

The full length of the human CDH6 protein has 790 amino acids (SEQ ID NO: 167), wherein the extracellular region comprises EC1 (SEQ ID NO: 168), EC2 (SEQ ID NO: 169), EC3 (SEQ ID NO: 170), EC4 (SEQ ID NO: 171), and EC5 (SEQ ID NO: 172). To confirm the binding epitope of the CDH6 antibody, the sequences after removal of these 5 regions were constructed into pcDNA3.1 vectors (purchased from Youbio, Cat. No. VT1001), and transfected into HEK293T cells (purchased from Cell Bank of Chinese Academy of Sciences, Cat. No. SCSP-502) to obtain 5 CDH6 truncated cell lines: HEK293T-hCDH6-EC1 deletion, HEK293T-hCDH6-EC2 deletion, HEK293T-hCDH6-EC3 deletion, HEK293T-hCDH6-EC4 deletion, and HEK293T-hCDH6-EC5 deletion.
Full-length sequence of human CDH6 protein (Uniprot# P55285) SEQ ID NO: 167 
MRTYRYFLLLFWVGQPYPTLSTPLSKRTSGFPAKKRALELSGNSKNELNRSKRSWMWNQF
FLLEEYTGSDYQYVGKLHSDQDRGDGSLKYILSGDGAGDLFIINENTGDIQATKRLDREEKP
VYILRAQAINRRTGRPVEPESEFIIKIHDINDNEPIFTKEVYTATVPEMSDVGTFVVQVTATDA
DDPTYGNSAKVVYSILQGQPYFSVESETGIIKTALLNMDRENREQYQVVIQAKDMGGQMG
GLSGTTTVNITLTDVNDNPPRFPQSTYQFKTPESSPPGTPIGRIKASDADVGENAEIEYSITDG
EGLDMFDVITDQETQEGIITVKKLLDFEKKKVYTLKVEASNPYVEPRFLYLGPFKDSATVRI
VVEDVDEPPVFSKLAYILQIREDAQINTTIGSVTAQDPDAARNPVKYSVDRHTDMDRIFNID
SGNGSIFTSKLLDRETLLWHNITVIATEINNPKQSSRVPLYIKVLDVNDNAPEFAEFYETFVCE
KAKADQLIQTLHAVDKDDPYSGHQFSFSLAPEAASGSNFTIQDNKDNTAGILTRKNGYNRH
EMSTYLLPVVISDNDYPVQSSTGTVTVRVCACDHHGNMQSCHAEALIHPTGLSTGALVAIL
LCIVILLVTVVLFAALRRQRKKEPLIISKEDIRDNIVSYNDEGGGEEDTQAFDIGTLRNPEAIE
DNKLRRDIVPEALFLPRRTPTARDNTDVRDFINQRLKENDTDPTAPPYDSLATYAYEGTGSV
ADSLSSLESVTTDADQDYDYLSDWGPRFKKLADMYGGVDSDKDS
EC1 SEQ ID NO: 168 
SWMWNQFFLLEEYTGSDYQYVGKLHSDQDRGDGSLKYILSGDGAGDLFIINENTGDIQAT
KRLDREEKPVYILRAQAINRRTGRPVEPESEFIIKIHDINDNEPIF
EC2 SEQ ID NO: 169 
TKEVYTATVPEMSDVGTFVVQVTATDADDPTYGNSAKVVYSILQGQPYFSVESETGIIKTAL
LNMDRENREQYQVVIQAKDMGGQMGGLSGTTTVNITLTDVNDNPPRF
EC3 SEQ ID NO: 170 
PQSTYQFKTPESSPPGTPIGRIKASDADVGENAEIEYSITDGEGLDMFDVITDQETQEGIITVK
KLLDFEKKKVYTLKVEASNPYVEPRFLYLGPFKDSATVRIVVEDVDEPPVF
EC4 SEQ ID NO: 171 
SKLAYILQIREDAQINTTIGSVTAQDPDAARNPVKYSVDRHTDMDRIFNIDSGNGSIFTSKLL
DRETLLWHNITVIATEINNPKQSSRVPLYIKVLDVNDNAP
EC5 SEQ ID NO: 172

EFAEFYETFVCEKAKADQLIQTLHAVDKDDPYSGHQFSFSLAPEAASGSNFTIQDNKDNTA
GILTRKNGYNRHEMSTYLLPVVISDNDYPVQSSTGTVTVRVCACDHHGNMQSCHAEALIH
P

The binding activity of the CDH6 chimeric antibodies to these 5 cell lines was assayed by FACS. The results are shown in Table 21. Based on the assay results, the binding epitopes of the antibodies can be presumed: CHI-CDH6-1 and CHI-CDH6-7 do not bind to EC1-deletion cells, indicating that their binding epitope is EC1; CHI-CDH6-5 does not bind to EC1-deletion cells and binds to EC2-deletion cells very weakly, indicating that its binding epitope may be between EC1-EC2; CHI-CDH6-4 does not bind to EC2-deletion cells, indicating that its binding epitope is EC2; CHI-CDH6-14 binds to EC2-deletion and EC3-deletion cells very weakly, indicating that its binding epitope may be between EC2-EC3; CHI-CDH6-3 binds to EC3-deletion cells very weakly, indicating that its binding epitope is EC3; CHI-CDH6-12 does not bind to EC3-deletion cells, indicating that its binding epitope is EC3.

**Table 21. Binding epitope of CDH6 chimeric antibodies to human CDH6**

| Antibody | EC1-deletion | EC2-deletion | EC3-deletion | EC4-deletion | EC5-deletion | Epitope |
|---|---|---|---|---|---|---|
| | Max MFI | Max MFI | Max MFI | Max MFI | Max MFI | |
| CHI-CDH6-1 | 84 | 51290 | 18022 | 29987 | 14345 | EC1 |
| CHI-CDH6-3 | 11076 | 7554 | 689 | 16799 | 5608 | EC3 |
| CHI-CDH6-4 | 10219 | 75 | 15097 | 22153 | 6543 | EC2 |
| CHI-CDH6-5 | 112 | 343 | 12177 | 18730 | 4244 | EC1-EC2 |
| CHI-CDH6-7 | 88 | 13037 | 9060 | 17357 | 1025 | EC1 |
| CHI-CDH6-12 | 9857 | 10971 | 70 | 16580 | 4488 | EC3 |
| CHI-CDH6-14 | 7211 | 814 | 755 | 26635 | 4038 | EC2-EC3 |
| DS-H01L02 | 5292 | 8387 | 62 | 11848 | 1528 | EC3 |
| NOV0712 | 23565 | 39127 | 16317 | 20571 | 78 | EC5 |
| hIgG1 | 83 | 61 | 65 | 59 | 81 | / |

| | | | | | | |
|---|---|---|---|---|---|---|
| Note: MFI ~1.5 or more times higher than that of the negative control hIgG1 can be considered binding. | | | | | | |

## Claims

1. An antibody or an antigen-binding fragment specifically binding to CDH6, wherein the antibody or the antigen-binding fragment specifically binds to one or more amino acid sequence fragments of EC1 region, EC1-EC2 region, EC2 region, EC2-EC3 region, EC3 region, EC4 region, or EC5 region of an extracellular region of CDH6 protein; preferably, the antibody or the antigen-binding fragment specifically binds to one or more amino acid sequence fragments set forth in any one sequence of SEQ ID NOs: 168-172; preferably, the antibody or the antigen-binding fragment has endocytic activity.

2. An antibody or an antigen-binding fragment specifically binding to CDH6, wherein the antibody or the antigen-binding fragment comprises:
(a) an HCDR1, an HCDR2, and an HCDR3 of the VH set forth in any one of SEQ ID NOs: 2, 4, 6, 8, 10, 12, 14, 16, 18, 20, 22, 24, 26, 28, 136, 137, 138, 139, 150, 151, 152, 160, 161, 162, 163, 164, and 165, and/or (b) an LCDR1, an LCDR2, and an LCDR3 of the VL set forth in any one of SEQ ID NOs: 3, 5, 7, 9, 11, 13, 15, 17, 19, 21, 23, 25, 27, 29, 133, 134, 135, 145, 146, 147, 148, 149, 157, 158, and 159;
preferably, the HCDR1-3 and/or the LCDR1-3 are encoded according to a universal analysis method of KABAT, IMGT, or Chothia.

3. The antibody or the antigen-binding fragment according to claim 2, wherein the HCDR1, the HCDR2 and the HCDR3 have a sequence combination selected from any of the following sequence combinations or a sequence combination with 1, 2, 3, or more amino acid insertions, deletions, and/or substitutions compared with the sequence combinations:
| Sequence No. | SEQ ID NO. | | |
|---|---|---|---|
| | HCDR1 | HCDR2 | HCDR3 |
| VH1 | SEQ ID NO.36 | SEQ ID NO.37 | SEQ ID NO.38 |
| VH2 | SEQ ID NO.42 | SEQ ID NO.43 | SEQ ID NO.44 |
| VH3 | SEQ ID NO.48 | SEQ ID NO.49 | SEQ ID NO.50 |
| VH4 | SEQ ID NO.54 | SEQ ID NO.55 | SEQ ID NO.56 |
| VH5 | SEQ ID NO.60 | SEQ ID NO.61 | SEQ ID NO.62 |
| VH6 | SEQ ID NO.66 | SEQ ID NO.67 | SEQ ID NO.68 |
| VH7 | SEQ ID NO.72 | SEQ ID NO.73 | SEQ ID NO.74 |
| VH8 | SEQ ID NO.78 | SEQ ID NO.79 | SEQ ID NO.80 |
| VH9 | SEQ ID NO.84 | SEQ ID NO.85 | SEQ ID NO.86 |
| VH10 | SEQ ID NO.90 | SEQ ID NO.91 | SEQ ID NO.92 |
| VH11 | SEQ ID NO.96 | SEQ ID NO.97 | SEQ ID NO.98 |
| VH12 | SEQ ID NO.102 | SEQ ID NO.103 | SEQ ID NO.104 |
| VH13 | SEQ ID NO.108 | SEQ ID NO.109 | SEQ ID NO.110 |
| VH14 | SEQ ID NO.114 | SEQ ID NO.115 | SEQ ID NO.116 |
and,
the LCDR1, the LCDR2 and the LCDR3 have a sequence combination selected from any of the following sequence combinations or a sequence combination with 1, 2, 3, or more amino acid insertions, deletions, and/or substitutions compared with the sequence combinations:
| Sequence No. | SEQ ID NO. | | |
|---|---|---|---|
| | LCDR1 | LCDR2 | LCDR3 |
| VL1 | SEQ ID NO.39 | SEQ ID NO.40 | SEQ ID NO.41 |
| VL2 | SEQ ID NO.45 | SEQ ID NO.46 | SEQ ID NO.47 |
| VL3 | SEQ ID NO.51 | SEQ ID NO.52 | SEQ ID NO.53 |
| VL4 | SEQ ID NO.57 | SEQ ID NO.58 | SEQ ID NO.59 |
| VL5 | SEQ ID NO.63 | SEQ ID NO.64 | SEQ ID NO.65 |
| VL6 | SEQ ID NO.69 | SEQ ID NO.70 | SEQ ID NO.71 |
| VL7 | SEQ ID NO.75 | SEQ ID NO.76 | SEQ ID NO.77 |
| VL8 | SEQ ID NO.81 | SEQ ID NO.82 | SEQ ID NO.83 |
| VL9 | SEQ ID NO.87 | SEQ ID NO.88 | SEQ ID NO.89 |
| VL10 | SEQ ID NO.93 | SEQ ID NO.94 | SEQ ID NO.95 |
| VL11 | SEQ ID NO.99 | SEQ ID NO.100 | SEQ ID NO.101 |
| VL12 | SEQ ID NO.105 | SEQ ID NO.106 | SEQ ID NO.107 |
| VL13 | SEQ ID NO.111 | SEQ ID NO.112 | SEQ ID NO.113 |
| VL14 | SEQ ID NO.117 | SEQ ID NO.118 | SEQ ID NO.119 |
preferably, the substitutions are conservative amino acid substitutions.

4. The antibody or the antigen-binding fragment according to claim 3, comprising a combination of heavy chain CDRs and light chain CDRs selected from: VH1+VL1, VH2+VL2, VH3+VL3, VH4+VL4, VH5+VL5, VH6+VL6, VH7+VL7, VH8+VL8, VH9+VL9, VH10+VL10, VH11+VL11, VH12+VL12, VH13+VL13, and VH14+VL14, and a combination of CDRs with 1, 2, 3 or more amino acid insertions, deletions, and/or substitutions compared with the sequences of the combination of the heavy chain CDRs and the light chain CDRs, wherein
preferably, the substitutions are conservative amino acid substitutions.

5. The antibody or the antigen-binding fragment according to any one of claims 1-4, wherein framework regions of the heavy chain variable region and the light chain variable region of the antibody or the antigen-binding fragment are derived from a human germline heavy chain template and a human germline light chain template, wherein:
(1) the framework region sequences are derived from a combined sequence of a human germline heavy chain IGHV1-69*02 and IGHJ6*01, comprising FR1, FR2, and FR3 regions of IGHV1-69*02 set forth in SEQ ID NO: 143 and an FR4 region of IGHJ6*01 set forth in SEQ ID NO: 144;
(2) the framework region sequences are derived from a combined sequence of a human germline heavy chain IGHV1-46*01 and IGHJ6*01, comprising FR1, FR2, and FR3 regions of IGHV1-46*01 set forth in SEQ ID NO: 156 and an FR4 region of IGHJ6*01 set forth in SEQ ID NO: 144;
(3) the framework region sequences are derived from a combined sequence of a human germline heavy chain IGHV1-3*01 and IGHJ6*01, comprising FR1, FR2, and FR3 regions of IGHV1-3*01 set forth in SEQ ID NO: 166 and an FR4 region of IGHJ6*01 set forth in SEQ ID NO: 144;
(4) the framework region sequences are derived from a combined sequence of a human germline light chain IGKV4-1*01 and IGKJ4*01, comprising FR1, FR2, and FR3 regions of IGKV4-1 *01 set forth in SEQ ID NO: 140 and an FR4 region of IGKJ4*01 set forth in SEQ ID NO: 142;
(5) the framework region sequences are derived from a combined sequence of a human germline light chain IGKV1-33*01 and IGKJ4*01, comprising FR1, FR2, and FR3 regions of IGKV1-33*01 set forth in SEQ ID NO: 141 and an FR4 region of IGKJ4*01 set forth in SEQ ID NO: 142;
(6) the framework region sequences are derived from a combined sequence of a human germline light chain IGKV1-NL1*01 and IGKJ2*01, comprising FR1, FR2, and FR3 regions of IGKV1-NL1*01 set forth in SEQ ID NO: 153 and an FR4 region of IGKJ2*01 set forth in SEQ ID NO: 155; or
(7) the framework region sequences are derived from a combined sequence of a human germline light chain IGKV2-28*01 and IGKJ2*01, comprising FR1, FR2, and FR3 regions of IGKV2-28*01 set forth in SEQ ID NO: 154 and an FR4 region of IGKJ2*01 set forth in SEQ ID NO: 155.

6. The antibody or the antigen-binding fragment according to claim 5, wherein according to numbers of the Kabat numbering scheme, the framework regions of the heavy chain variable region and the light chain variable region of the antibody or the antigen-binding fragment further comprise one or more mutations selected from the following groups, wherein
(1) the framework regions of the heavy chain variable region comprise: G27Y, S30T, A40R, I70L, or A72V, preferably comprise G27Y and A72V, or preferably comprise G27Y, S30T, and A72V, or preferably comprise G27Y, S30T, I70L, and A72V, or preferably comprise G27Y, S30T, A40R, and A72V;
(2) the framework regions of the heavy chain variable region comprise: G42R, M70L, R72V, or T74K, preferably comprise R72V and T74K, or preferably comprise M70L, R72V, and T74K, or preferably comprise G42R, R72V, and T74K;
(3) the framework regions of the heavy chain variable region comprise: V2I, V5Q, R44G, I70L, R72V, Y95L, or R98S, preferably comprise R72V and R98S, or preferably comprise V2I, R72V, and R98S, or preferably comprise V2I, I70L, R72V, and R98S, or preferably comprise R44G, R72V, and R98S, or preferably comprise V5Q, R72V, and R98S, or preferably comprise R72V, Y95L, and R98S;
(4) the framework regions of the light chain variable region comprise: P44S, A47P, or G72E, preferably comprise G72E, or preferably comprise P44S and G72E, or preferably comprise A47P and G72E;
(5) the framework regions of the light chain variable region comprise: Q42K, A43S, K45Q, L48V, I48V, or T85R, preferably comprise A43S, or preferably comprise A43S and L48V, or preferably comprise A43S, K45Q, and L48V, or preferably comprise A43S, L48V, and T85R, or preferably comprise Q42K and I48V; or
(6) the framework regions of the light chain variable region comprise: K42G, P43T, P44F, or Y49S, preferably comprise P44F and Y49S, or preferably comprise K42G and Y49S, or preferably comprise P43T and Y49S.

7. The antibody or the antigen-binding fragment according to any one of claims 1-6, wherein the antibody or the antigen-binding fragment comprises:
(1) a heavy chain variable region having the sequence set forth in SEQ ID NO: 2, 4, 6, 8, 10, 12, 14, 16, 18, 20, 22, 24, 26, 28, 136, 137, 138, 139, 150, 151, 152, 160, 161, 162, 163, 164, or 165;
(2) a light chain variable region having the sequence set forth in SEQ ID NO: 3, 5, 7, 9, 11, 13, 15, 17, 19, 21, 23, 25, 27, 29, 133, 134, 135, 145, 146, 147, 148, 149, 157, 158, or 159;
(3) an amino acid sequence having at least 90% identity, preferably at least 91%, 92%, 93%, 94%, 95%, 96%, 97%, 98%, or 99% identity, to any one of the sequences set forth in (1) to (2) above.

8. The antibody or the antigen-binding fragment according to claim 7, wherein the heavy chain variable region and the light chain variable region are selected from the following groups:
(1) having the VH set forth in SEQ ID NO. 2 and the VL set forth in SEQ ID NO. 3;
(2) having the VH set forth in SEQ ID NO. 4 and the VL set forth in SEQ ID NO. 5;
(3) having the VH set forth in SEQ ID NO. 6 and the VL set forth in SEQ ID NO. 7;
(4) having the VH set forth in SEQ ID NO. 8 and the VL set forth in SEQ ID NO. 9;
(5) having the VH set forth in SEQ ID NO. 10 and the VL set forth in SEQ ID NO. 11;
(6) having the VH set forth in SEQ ID NO. 12 and the VL set forth in SEQ ID NO. 13;
(7) having the VH set forth in SEQ ID NO. 14 and the VL set forth in SEQ ID NO. 15;
(8) having the VH set forth in SEQ ID NO. 16 and the VL set forth in SEQ ID NO. 17;
(9) having the VH set forth in SEQ ID NO. 18 and the VL set forth in SEQ ID NO. 19;
(10) having the VH set forth in SEQ ID NO. 20 and the VL set forth in SEQ ID NO. 21;
(11) having the VH set forth in SEQ ID NO. 22 and the VL set forth in SEQ ID NO. 23;
(12) having the VH set forth in SEQ ID NO. 24 and the VL set forth in SEQ ID NO. 25;
(13) having the VH set forth in SEQ ID NO. 26 and the VL set forth in SEQ ID NO. 27;
(14) having the VH set forth in SEQ ID NO. 28 and the VL set forth in SEQ ID NO. 29;
(15) having the VH set forth in SEQ ID NO. 136 and the VL set forth in SEQ ID NO. 133;
(16) having the VH set forth in SEQ ID NO. 137 and the VL set forth in SEQ ID NO. 133;
(17) having the VH set forth in SEQ ID NO. 138 and the VL set forth in SEQ ID NO. 133;
(18) having the VH set forth in SEQ ID NO. 139 and the VL set forth in SEQ ID NO. 133;
(19) having the VH set forth in SEQ ID NO. 136 and the VL set forth in SEQ ID NO. 134;
(20) having the VH set forth in SEQ ID NO. 137 and the VL set forth in SEQ ID NO. 134;
(21) having the VH set forth in SEQ ID NO. 138 and the VL set forth in SEQ ID NO. 134;
(22) having the VH set forth in SEQ ID NO. 139 and the VL set forth in SEQ ID NO. 134;
(23) having the VH set forth in SEQ ID NO. 136 and the VL set forth in SEQ ID NO. 135;
(24) having the VH set forth in SEQ ID NO. 137 and the VL set forth in SEQ ID NO. 135;
(25) having the VH set forth in SEQ ID NO. 138 and the VL set forth in SEQ ID NO. 135;
(26) having the VH set forth in SEQ ID NO. 139 and the VL set forth in SEQ ID NO. 135;
(27) having the VH set forth in SEQ ID NO. 150 and the VL set forth in SEQ ID NO. 145;
(28) having the VH set forth in SEQ ID NO. 150 and the VL set forth in SEQ ID NO. 146;
(29) having the VH set forth in SEQ ID NO. 150 and the VL set forth in SEQ ID NO. 147;
(30) having the VH set forth in SEQ ID NO. 150 and the VL set forth in SEQ ID NO. 148;
(31) having the VH set forth in SEQ ID NO. 150 and the VL set forth in SEQ ID NO. 149;
(32) having the VH set forth in SEQ ID NO. 151 and the VL set forth in SEQ ID NO. 145;
(33) having the VH set forth in SEQ ID NO. 151 and the VL set forth in SEQ ID NO. 146;
(34) having the VH set forth in SEQ ID NO. 151 and the VL set forth in SEQ ID NO. 147;
(35) having the VH set forth in SEQ ID NO. 151 and the VL set forth in SEQ ID NO. 148;
(36) having the VH set forth in SEQ ID NO. 151 and the VL set forth in SEQ ID NO. 149;
(37) having the VH set forth in SEQ ID NO. 152 and the VL set forth in SEQ ID NO. 145;
(38) having the VH set forth in SEQ ID NO. 152 and the VL set forth in SEQ ID NO. 146;
(39) having the VH set forth in SEQ ID NO. 152 and the VL set forth in SEQ ID NO. 147;
(40) having the VH set forth in SEQ ID NO. 152 and the VL set forth in SEQ ID NO. 148;
(41) having the VH set forth in SEQ ID NO. 152 and the VL set forth in SEQ ID NO. 149;
(42) having the VH set forth in SEQ ID NO. 160 and the VL set forth in SEQ ID NO. 157;
(43) having the VH set forth in SEQ ID NO. 160 and the VL set forth in SEQ ID NO. 158;
(44) having the VH set forth in SEQ ID NO. 160 and the VL set forth in SEQ ID NO. 159;
(45) having the VH set forth in SEQ ID NO. 161 and the VL set forth in SEQ ID NO. 157;
(46) having the VH set forth in SEQ ID NO. 161 and the VL set forth in SEQ ID NO. 158;
(47) having the VH set forth in SEQ ID NO. 161 and the VL set forth in SEQ ID NO. 159;
(48) having the VH set forth in SEQ ID NO. 162 and the VL set forth in SEQ ID NO. 157;
(49) having the VH set forth in SEQ ID NO. 162 and the VL set forth in SEQ ID NO. 158;
(50) having the VH set forth in SEQ ID NO. 162 and the VL set forth in SEQ ID NO. 159;
(51) having the VH set forth in SEQ ID NO. 163 and the VL set forth in SEQ ID NO. 157;
(52) having the VH set forth in SEQ ID NO. 163 and the VL set forth in SEQ ID NO. 158;
(53) having the VH set forth in SEQ ID NO. 163 and the VL set forth in SEQ ID NO. 159;
(54) having the VH set forth in SEQ ID NO. 164 and the VL set forth in SEQ ID NO. 157;
(55) having the VH set forth in SEQ ID NO. 164 and the VL set forth in SEQ ID NO. 158;
(56) having the VH set forth in SEQ ID NO. 164 and the VL set forth in SEQ ID NO. 159;
(57) having the VH set forth in SEQ ID NO. 165 and the VL set forth in SEQ ID NO. 157;
(58) having the VH set forth in SEQ ID NO. 165 and the VL set forth in SEQ ID NO. 158;
(59) having the VH set forth in SEQ ID NO. 165 and the VL set forth in SEQ ID NO. 159; or
(60) a combination of a VH and a VL having at least 90%, 91%, 92%, 93%, 94%, 95%, 96%, 97%, 98%, 99%, or 100% sequence identity to any one of the sequence combinations (1) to (59) above.

9. The antibody or the antigen-binding fragment according to any one of claims 1-8, wherein the antibody or the antigen-binding fragment binds to human CDH6 protein with a dissociation constant (KD) not greater than 2×10⁻⁷ M.

10. The antibody or the antigen-binding fragment according to any one of claims 1-9, wherein the antibody or the antigen-binding fragment is:
(1) a chimeric antibody or a fragment thereof;
(2) a humanized antibody or a fragment thereof;
(3) a fully human antibody or a fragment thereof;
preferably, the antibody or the antigen-binding fragment is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', a F(ab')2, an Fd, an Fv, an scFv, a diabody, and a single domain antibody.

11. The antibody or the antigen-binding fragment according to any one of claims 1-10, wherein the antibody comprises a sequence of a constant region of any one of human or murine antibodies IgG1, IgG2, IgG3, IgG4, IgA, IgM, IgE, and IgD, and preferably comprises a sequence of a constant region of human or murine antibody IgG1, IgG2, IgG3, or IgG4.

12. The antibody or the antigen-binding fragment according to any one of claims 1-11, wherein the antigen-binding fragment is selected from one or more of a F(ab)2, a Fab', a Fab, an Fv, an scFv, a bispecific antibody, a nanobody, and a minimal recognition unit of an antibody.

13. The antibody or the antigen-binding fragment according to any one of claims 1-12, wherein the antibody or the antigen-binding fragment is further conjugated to a therapeutic agent or tracer, wherein preferably, the therapeutic agent is selected from a radioisotope, a chemotherapeutic agent, and an immunomodulator, and the tracer is selected from a radiocontrast medium, a paramagnetic ion, a metal, a fluorescent label, a chemiluminescent label, an ultrasound contrast agent, and a photosensitizer.

14. A multispecific antigen-binding molecule, comprising a first antigen-binding moiety and a second antigen-binding moiety, wherein the first antigen-binding moiety comprises the antibody or the antigen-binding fragment according to any one of claims 1-13, and the second antigen-binding moiety specifically binds to an antigen other than CDH6 or to a CDH6 epitope different from the first antigen-binding moiety, wherein
preferably, the antigen is selected from CD3, CD7, CD16, CD16A, CD4, CD5, CD8, CD14, CD15, CD19, CD20, CD21, CD23, CD25, CD33, CD37, CD38, CD40, CD40L, CD46, CD52, CD54, CD66(a-d), CD74, CD80, CD126, CD138, BCMA, HLA-DR, HER2, VEGF, P1GF, HER3/ERBB3, HER4/ERBB4, IL-2, IL-6, PD-1, PD-L1, TRAIL-R1, and TRAIL-R2;
preferably, the multispecific antigen-binding molecule is a bispecific antibody, a trispecific antibody, or a tetraspecific antibody.

15. A chimeric antigen receptor (CAR), at least comprising an extracellular antigen-binding domain, a transmembrane domain, and an intracellular signaling domain, wherein the extracellular antigen-binding domain comprises the CDH6 antibody or the antigen-binding fragment according to any one of claims 1-13.

16. An immune effector cell, comprising the chimeric antigen receptor according to claim 15 or a nucleic acid fragment encoding the chimeric antigen receptor according to claim 15, wherein
preferably, the immune effector cell is selected from a T cell, an NK cell (natural killer cell), an NKT cell (natural killer T cell), a monocyte, a macrophage, a dendritic cell, and a mast cell, wherein the T cell can be selected from an inflammatory T cell, a cytotoxic T cell, a regulatory T cell (Treg), and a helper T cell; and
preferably, the immune effector cell is an allogeneic immune effector cell or an autologous immune cell.

17. An isolated nucleic acid molecule, wherein the nucleic acid molecule encodes the antibody or the antigen-binding fragment according to any one of claims 1-13 or any combination thereof, the multispecific antigen-binding molecule according to claim 14, or the chimeric antigen receptor according to claim 15.

18. An expression vector, comprising the isolated nucleic acid molecule according to claim 17.

19. An isolated host cell, comprising the isolated nucleic acid molecule according to claim 17 or the expression vector according to claim 18, wherein preferably, the host cell is a eukaryotic cell or a prokaryotic cell; more preferably, the host cell is derived from a mammalian cell, a yeast cell, an insect cell, *E. coli,* and/or *Bacillus subtilis;* and more preferably, the host cell is selected from Expi293 and CHO cells.

20. A method for preparing the antibody or the antigen-binding fragment according to any one of claims 1-13 or the multispecific antigen-binding molecule according to claim 14, comprising culturing the host cell according to claim 19 under appropriate conditions, and isolating the antibody or the antigen-binding fragment or the multispecific antigen-binding molecule.

21. A method for preparing the immune effector cell according to claim 16, comprising introducing a nucleic acid fragment encoding the chimeric antigen receptor according to claim 15 into the immune effector cell, wherein optionally, the method further comprises initiating expression of the chimeric antigen receptor according to claim 15 in the immune effector cell.

22. A pharmaceutical composition, comprising the antibody or the antigen-binding fragment according to any one of claims 1-13, the multispecific antigen-binding molecule according to claim 14, the chimeric antigen receptor according to claim 15, the immune effector cell according to claim 16, the isolated nucleic acid molecule according to claim 17, the expression vector according to claim 18, the cell according to claim 19, or a product prepared by the method according to claim 20 or 21, wherein preferably, the composition further comprises a pharmaceutically acceptable carrier, diluent, or adjuvant; preferably, the pharmaceutical composition further comprises an additional antineoplastic agent.

23. Use of the antibody or the antigen-binding fragment according to any one of claims 1-13, the multispecific antigen-binding molecule according to claim 14, the chimeric antigen receptor according to claim 15, the immune effector cell according to claim 16, the isolated nucleic acid molecule according to claim 17, the expression vector according to claim 18, the cell according to claim 19, a product prepared by the method according to claim 20 or 21, or the pharmaceutical composition according to claim 22 in preparing a medicament for preventing and/or treating a tumor disease, wherein
preferably, the tumor disease is selected from a solid tumor, wherein preferably, the solid tumor is a solid tumor expressing CDH6 protein; more preferably, the solid tumor is selected from kidney cancer, ovarian cancer, thyroid cancer, cholangiocarcinoma, lung cancer, glioblastoma, mesothelioma, uterine cancer, pancreatic cancer, Wilms tumor, and neuroblastoma.

24. A method for preventing and/or treating a tumor disease, comprising administering to a patient in need thereof an effective amount of the antibody or the antigen-binding fragment according to any one of claims 1-13, the multispecific antigen-binding molecule according to claim 14, the chimeric antigen receptor according to claim 15, the immune effector cell according to claim 16, the isolated nucleic acid molecule according to claim 17, the expression vector according to claim 18, the cell according to claim 19, a product prepared by the method according to claim 20 or 21, or the pharmaceutical composition according to claim 22, wherein
preferably, the tumor disease is selected from a solid tumor, wherein preferably, the solid tumor is a solid tumor expressing CDH6 protein; more preferably, the solid tumor is selected from kidney cancer, ovarian cancer, thyroid cancer, cholangiocarcinoma, lung cancer, glioblastoma, mesothelioma, uterine cancer, pancreatic cancer, Wilms tumor, and neuroblastoma.

25. The antibody or the antigen-binding fragment according to any one of claims 1-13, the multispecific antigen-binding molecule according to claim 14, the chimeric antigen receptor according to claim 15, the immune effector cell according to claim 16, the isolated nucleic acid molecule according to claim 17, the expression vector according to claim 18, the cell according to claim 19, a product prepared by the method according to claim 20 or 21, or the pharmaceutical composition according to claim 22 for use in preventing and/or treating a tumor disease, wherein
preferably, the tumor disease is selected from a solid tumor, wherein preferably, the solid tumor is a solid tumor expressing CDH6 protein; more preferably, the solid tumor is selected from kidney cancer, ovarian cancer, thyroid cancer, cholangiocarcinoma, lung cancer, glioblastoma, mesothelioma, uterine cancer, pancreatic cancer, Wilms tumor, and neuroblastoma.

26. A kit, comprising the antibody or the antigen-binding fragment thereof according to any one of claims 1-13, the multispecific antigen-binding molecule according to claim 14, the chimeric antigen receptor according to claim 15, the immune effector cell according to claim 16, the isolated nucleic acid molecule according to claim 17, the expression vector according to claim 18, the cell according to claim 19, a product prepared by the method according to claim 20 or 21, or the pharmaceutical composition according to claim 22, and optionally comprising instructions.
